(19) Europäisches Patentamt
European Patent Office
Office européen des brevets

(11) Publication number: **0 454 580 B1**

(12) **EUROPEAN PATENT SPECIFICATION**

(45) Date of publication of patent specification: **27.12.95**

(51) Int. Cl.[6]: **C07F 7/08**, A61K 31/695, C07F 7/18

(21) Application number: **91401096.2**

(22) Date of filing: **25.04.91**

(54) **Novel nojirimycin derivatives**

(30) Priority: **27.04.90 EP 90401169**

(43) Date of publication of application: **30.10.91 Bulletin 91/44**

(45) Publication of the grant of the patent: **27.12.95 Bulletin 95/52**

(84) Designated Contracting States: **AT BE CH DE DK ES FR GB GR IT LI LU NL SE**

(56) References cited:
**EP-A- 0 282 168**
**EP-A- 0 350 012**

(73) Proprietor: **MERRELL DOW PHARMACEUTICALS INC.**
**2110 East Galbraith Road,**
**P.O. Box 156300**
**Cincinnati**
**Ohio 45215-6300 (US)**

(72) Inventor: **Lesur, Brigitte**
**3, rue Klein**
**F-67000 Strasbourg (FR)**
Inventor: **Ducep, Jean-Bernard**
**29, rue des Alpes**
**F-68280 Sundhoffen (FR)**
Inventor: **Danzin, Charles**
**18, rue Geiler**
**F-67000 Strasbourg (FR)**

(74) Representative: **Gillard, Marie-Louise et al**
**Cabinet Beau de Loménie**
**158, rue de l'Université**
**F-75340 Paris Cédex 07 (FR)**

EP 0 454 580 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel N-derivatives of 1-deoxy nojirimycin, to the method for their preparation and to their use in the treatment of diabetes and their use against retro-viruses, particularly in the treatment of acquired immuno-deficiency syndrome (AIDS).

More specifically the invention relates to 1-deoxy nojirimycin derivatives of the formula

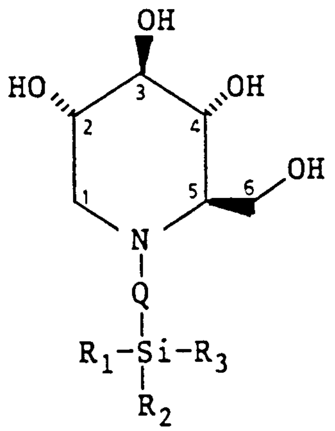

I

the geometric isomers thereof, and the pharmaceutically acceptable salts thereof wherein

| | |
|---|---|
| Q | is $C_{1-7}$ alkylene, $(CH_2)_mCH=CH(CH_2)n$, $(CH_2)_mC{\equiv}C(CH_2)n$, $(CH_2)_mCH=C=CH(CH_2)_n$, $(CH_2)_p$ phenylene, $(CH_2)_m$ cyclopentenylene, $(CH_2)_m$ cyclohexenylene, $(CH_2)_pT$, wherein T, together with the silicon atom to which it is attached forms a 5 to 6 atom cyclic silicane, said cyclic silicane optionally having a double bond, with m being 1, 2 or 3, n being 0, 1 or 2, p being 0, 1, 2, 3 or 4, |
| $R_1$ | is $C_{1-7}$ alkyl, $C_{1-7}$ alkoxy, mono- or polyhydroxy $C_{1-6}$ alkyl, mono- or polyalkoxy $C_{1-6}$ alkyl, chloro $C_{1-6}$ alkyl, |
| $R_2$ and $R_3$ | are $C_{1-10}$ alkyl, $(CH_2)_p$-X,Y-substituted phenyl, with X and Y each being H, OH, halogen, $C_{1-6}$ alkyl, $C_{1-6}$ alkoxy, $CF_3$, CN, $NO_2$, SH or -S-$C_{1-6}$ alkyl, with the proviso that when Q is $(CH_2)_pT$, then one of $R_1$, $R_2$ or $R_3$ is deleted. |

As used herein, Q is a divalent moiety bridging the 1-deoxy-nojirimycin with the silicon atom to which Q is attached. In all instances the moieties of Q are directly attached to the nitrogen atom of the 1-deoxy nojirimycin. The $C_{1-7}$ alkylene moiety includes the straight, branched and cyclized manifestations of saturated lower aliphatic hydrocarbons including such alkylene moieties derived from alkyl radicals such as methyl, ethyl, n-propyl, isopropyl, n-butyl, cyclopropyl, pentyl, hexyl, cyclohexyl, cyclohexylmethyl, preferably n-butyl, n-propyl, methyl or ethyl, (yielding, of course such moieties as for example ($-CH_2-$), ($-CH_2-CH_2-$), ($-CH_2CH_2CH_2-$), ($-CH_2-(C_6H_{10})$} wherein the silicon is preferably attached at the meta-position of the cyclohexyl moiety, and the like). Preferably the moiety of $(CH_2)_mCH=CH(CH_2)_n$ is in its trans configuration, and preferably m is 1 and n is zero. Preferably, the $(CH_2)_m$ cyclopentenylene and cyclohexenylene moieties have their unsaturation at the carbon atom to which the silicon is attached

[e.g. 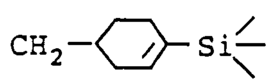 or 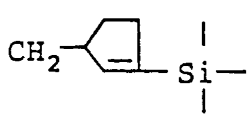 ].

Preferably, for the $(CH_2)_p$ phenylene moiety,p is 1 or 2 and the $-SiR_1R_2R_3$ moiety is attached to the phenyl moiety at its 3-position. Preferred $(CH_2)_pT$ moieties are illustrated as

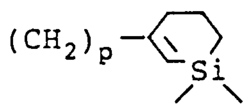 , 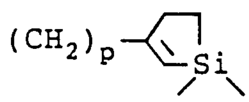 , 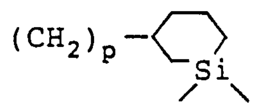 and

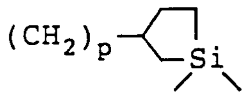

with p preferably being 1 or 2.

In those instances wherein $R_1$ is $C_{1-7}$ alkyl or $C_{1-7}$ alkoxy, methyl, ethyl, methoxy and ethoxy are preferred. When $R_1$ is hydroxy $C_{1-6}$ alkyl, hydroxymethyl is preferred. When $R_1$ is polyhydroxy $C_{1-6}$ alkyl it is preferred that there be 1, 2 or 3 OH radicals, each one of which is attached to a different carbon atom of the $C_{1-6}$ alkyl moiety and in such instances it is preferred that the hydroxy be located on carbon atoms other than the carbon atom attached directly onto the silicon atom; the same concepts also be true for the mono- and polyalkoxy substituted $C_{1-6}$ alkyl moieties in which case methoxy is preferred. Preferably the $(CH_2)_p$X,Y-substituted phenyl moieties are those wherein both X and Y are H, or one is H and the other is OH, chloro, methyl or methoxy or both are OH, Cl, methyl or methoxy. In general, in those instances wherein the Q radical contains a $(CH_2)_m$ moiety it is preferred that m be 1 or 2, and when Q contains $(CH_2)_n$ moieties n preferably is zero or 1. For convenience, the

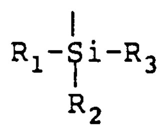

moiety and the

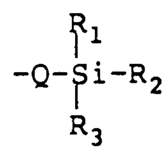

moiety of Formula I will also be referred to as $-SiR_1R_2R_3$ and $-Q-Si-R_1R_2R_3$, respectively. In general the trans- isomers are preferred over the cis- isomers.

The pharmaceutically acceptable salts of the compounds of formula I include salts formed with non-toxic organic or inorganic acids such as, for example, from the following acids: hydrochloric, hydrobromic, sulfonic, sulfuric, phosphoric, nitric, maleic, fumaric, benzoic, ascorbic, pamoic, succinic, methanesulfonic acid, acetic, propionic, tartaric, citric, lactic, malic, mandelic, cinnamic, palmitic, itaconic and benzenesulfonic and the like.

In general the compounds of this invention are prepared by chemical reactions and procedures which are analogously known in the art and the selection of a particular route to any specific compound is governed by principles well known and appreciated by those of ordinary skill in the art.

In general the compounds of this invention may be prepared according to the reaction scheme outlined below.

Reaction Scheme A

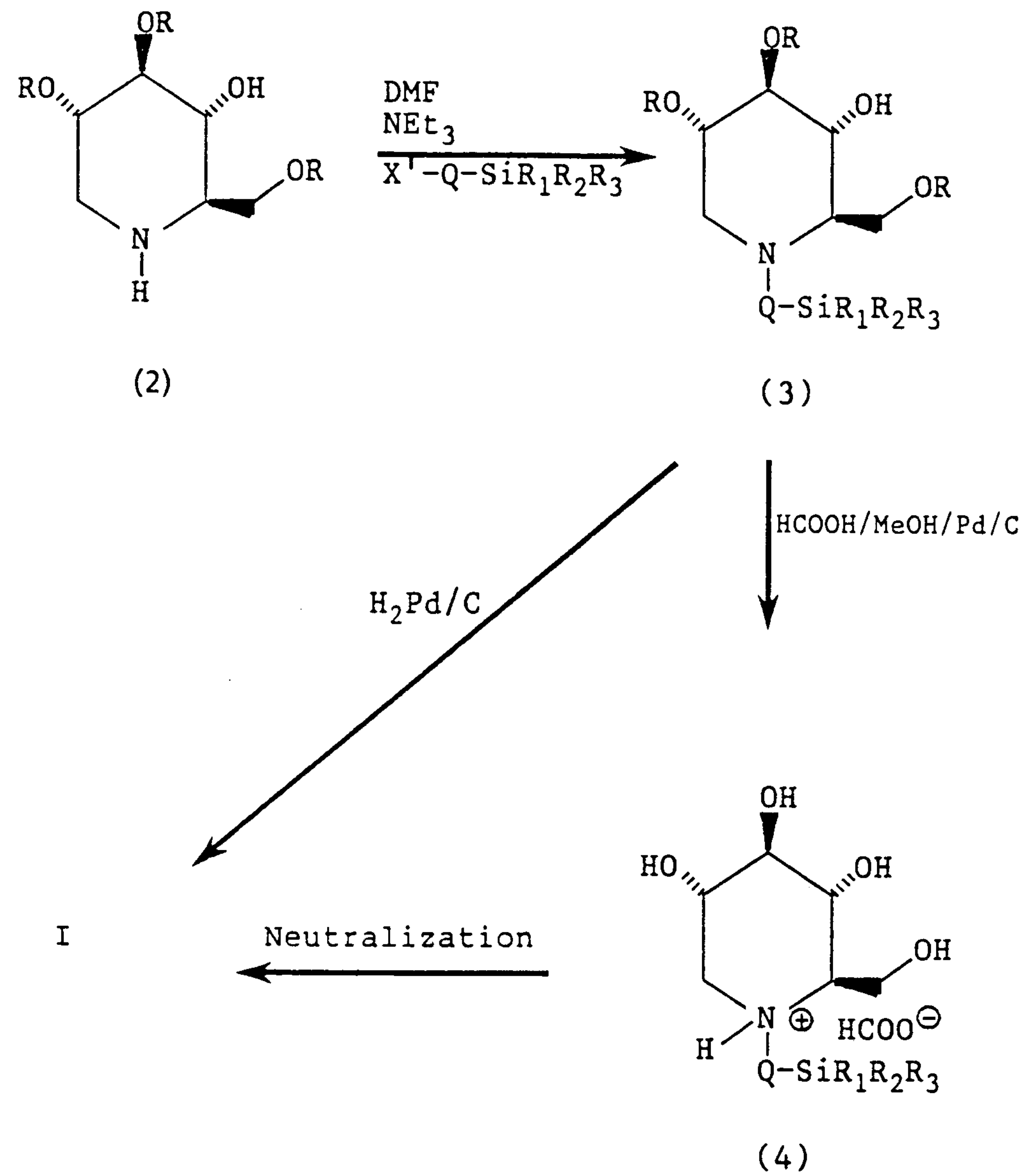


wherein Q-$SiR_1R_2R_3$ is as previously defined, R is H or Bz, Bz is benzyl, a preferred hydroxy-protecting group, X′ is halogeno, mesylate or tosylate.

The synthesis of Reaction Scheme A is initiated by the condensation of an optionally hydroxy-protected 1-deoxy nojirimycin with an excess quantity (≃ three times) of the appropriate X′-Q-$SiR_1R_2R_3$ reactant in the presence of an excess of triethylamine ($NEt_3$) in dimethyl formamide (DMF). Preferably X′ is the iodide. The so-produced compounds (3) are purified, preferably using chromatographic techniques and are then deprotected according to standard procedures well known in the art. Preferably deprotection is effected by using transfer hydrogenation with formic acid in methanol with Pd/C or by catalytic hydrogenation, preferably using palladium on carbon in an appropriate solvent, e.g. ethanol. When transfer hydrogenation is utilized, the deprotected products (4) are in the form of quaternary salts with the $HCOO^{\ominus}$ anion and thus must be neutralized; the neutralization preferably being effected using an ion exchange resin such as Dowex AX-8.

In those instances wherein Q represents a bridging moiety containing an unsaturation (e.g., allylic, allenic, acetylenic, cyclopentenylene, cyclohexenylene or the unsaturated $(CH_2)_pT$ moieties) it is preferred that the 2-, 3-and 6-position hydroxy groups not be protected in view of the difficulties encountered when removing the benzyl protecting groups unless special procedures are employed (e.g., when Q contains

such unsaturated moieties a process using sodium in ammonia is preferred). In those instances wherein Q is other than an unsaturated moiety, and it is preferred that the 2-, 3- and 6-positions bear a benzyl protecting group, "normal" procedures (as outlined above) may be employed for their removal. The benzyl groups may also serve as a means for obtaining the products in a more purified form. Although not required the 4-position OH group may also bear a protecting group.

It should be noted that when the above comment is made concerning the protection of the 2-, 3- and 6-OH functions, the nomenclature is that which is used in sugar chemistry using the positions of glucitol as shown in Formula I. In that instance, the OH radical of the hydroxymethyl moiety is at the 6-position, the other beta OH is at the 3-position and the two alpha OH groups are at the 2- and 4-positions. In that instance it is the 2-, 3- and 6-position hydroxy groups which would be protected prior to the condensation reaction (the 4-position OH group, in any situation, normally need not be protected). In the instances wherein the compounds are named as piperidine derivatives the compounds (1 and 4) would be 2($\beta$)-hydroxymethyl-1-[$R_1R_2R_3$Si-Q]-3$\alpha$,4$\beta$,5$\alpha$-piperidinetriols and thus for Structures 2 and 3 the OH functions at the 2-, 4- and 5-positions of the piperidine would be protected with a benzyl protecting group.

To illustrate the nomenclature of the compounds of this invention as piperidine derivatives the structured compound

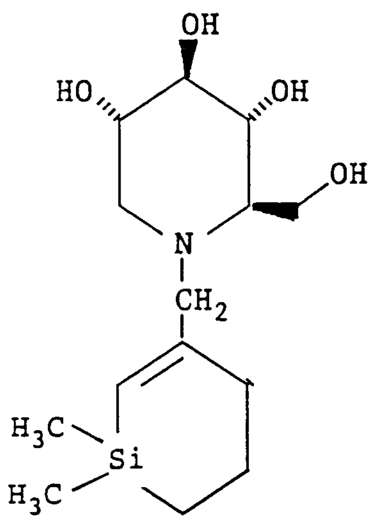

would be named [2R-(2$\beta$,3$\alpha$,4$\beta$,5$\alpha$]-2-hydroxymethyl-1-[1-(3,3-dimethyl-3-sila-1-cyclohexenyl)methyl]-3,4,5-piperidinetriol.

1-Deoxy nojirimycin may be obtained by reducing nojirimycin (5-amino-5-deoxy-D-glucopyranose) using the method of Tetrahedron Letters, 24, 2125-2144, 1968, as referenced in European Patent Application 89 112284.8 published on January 10, 1990, with publication No. 0350012. The preparation of 1-deoxy nojirimycin and its hydroxy-protected analogs (2) are also disclosed in the specific examples disclosed herein.

The X′-Q-Si$R_1R_2R_3$ reactant are either known compounds or may be prepared by methods analogously known in the art.

The following examples illustrate the preparation of the compounds of this invention.

EXAMPLE 1

Preparation of
3-(TRIMETHYLSILYL)-1-PROPANOL, METHANESULFONATE

Methanesulfonylchloride (0.73 ml, 9 mmol) was added dropwise to a solution of 3-(trimethylsilyl)-1-propanol (1.2 ml, 7.56 mmol) cooled at 0 °C in 20 ml of dichloro-methane. After 45 minutes stirring, the reaction mixture was partitioned between water and dichloromethane, the organic phase was separated, the solvent was evaporated under reduced pressure to afford the expected 3-(trimethylsilyl)-1-propanol, methanesulfonate in crude quantitative yield.

EXAMPLE 2

Preparation of
(3-IODOPROPYL)TRIMETHYSILANE

An ethereal solution of magnesium iodide was prepared by adding 4.85 g (19 mmol) of iodine to 0.46 g (19 mmol) of magnesium in suspension in 40 ml of dry ether. 28 ml of this solution was added to a solution containing the crude 3-(trimethylsilyl)-1-propanol, methanesulfonate in 10 ml of ether. The reaction mixture was stirred for 3 hours at room temperature and then partitioned between ether and water, the organic phase was separated and further washed with aqueous sodium thiosulfate. After evaporation of the solvent, 1.6 g of (3-iodopropyl)trimethysilane was obtained as a colorless liquid.

EXAMPLE 3

Preparation of
(3-BROMOPROPYL)TRIMETHYLSILANE

1.02 ml (10.9 mmol) of phosphorous tribromide in 20 ml of ether was added to a cooled (0 °C, -10 °C) solution of 4 g (30.2 mmol) of 3-(trimethylsilyl)-1-propanol in 40 ml of dry ether. The reaction mixture was brought back to room temperature and refluxed for 15 minutes. After bulb to bulb distillation of the crude reaction mixture 4.3g of (3-bromopropyl)trimethylsilanewere isolated as a colorless liquid.

EXAMPLE 4

Preparation of
4-(TRIMETHYLSILYL)-BUTANOIC ACID

A solution containing 3 g (15.4 mmol) of (3-bromopropyl)trimethylsilane in 3 ml of dry ether was added to 0.375 g (15.4 mmol) of magnesium turnings in ether (40 ml final volume of solution). After 1 hour of reflux gazeous carbondioxide was bubbled through the reaction mixture (3 g, 77 mmol of dry ice). After 2 hours of stirring at room temperature the reaction mixture was partitioned between aqueous ammonium chloride and ether. The organic phase was separated, the aqueous phase was further acidified with hydrochloric acid 1N and extracted with ether. Ethereal phases were pooled and the solvent was removed under reduced pressure. Separation of the expected 4-trimethylsilylbutanoic acid from dimer of starting (3-bromopropyl)-trimethylsilane was finally performed by acid base extraction. 0.62 g of 4-(trimethylsilyl)-butanoic acid was isolated as a colorless liquid.

EXAMPLE 5

Preparation of
4-(TRIMETHYLSILYL)-1-BUTANOL

1.5 ml (11.4 mmol) of a 1 molar solution of borane dimethylsulfide was added to a cooled (0 °C) solution of 0.61 g (3.8 mmol) of 4-(trimethylsilyl)-butanoic acid in 20 ml of dry tetrahydrofuran. After work-up using the standard procedure (methanol, tetramethylethylene diamine) and flash chromatography purification on silica gel eluted with a 9:1 mixture of hexane and ethyl acetate, 0.4 g of 4-(trimethylsilyl)-1-butanol was obtained as a colorless liquid.

EXAMPLE 6

Preparation of
4-(TRIMETHYLSILYL)-1-BUTANOL, METHANESULFONATE

Starting from 0.4 g (2.53 mmol) of 4-(trimethylsilyl)-1-butanol, 0.245 ml (3.16 mmol) of methanesulfonyl chloride and 0.528 ml (4.3 mmol) of triethylamine, and using the same procedure as for the preparation of 3-(trimethylsilyl)-1-propanol, methanesulfonate, 0.5 g of the expected 4-(trimethylsilyl)-1-butanol, methanesulfonate was obtained.

EXAMPLE 7

Preparation of 4-(IODOBUTYL)TRIMETHYLSILANE

Using the same procedure as described for the preparation of 3-(iodopropyl)trimethylsilane, starting from 0.5 g (2.53 mmol) of 4-(trimethylsilyl)-1-butanol methane sulfonate and 12 ml of a 0.34 M ethereal solution of magnesium iodider 0.5 g of 4-(iodobutyl)trimethylsilane was isolated as a colorless liquid.

EXAMPLE 8

Preparation of (4-BROMO-2-BUTYNYL)TRIMETHYLSILANE

4-(trimethylsilyl)-2-butynol [J. Pernet, B. Randrianoelina, and L. Miginiac, Tetrahedron Letters, 25, 651, (1984)] (10 g, 70 mmol) is dissolved in dry diethyl ether (150 ml) and phosphorous tribromide (2.2 ml, 23.3 mmol) is added dropwise. Then the mixture is refluxed, protected from the light during 2.5 hours. The reaction is washed twice with water, once with aqueous sodium bicarbonate and then once with water. The organic layer is dried over sodium sulfate. The solvent is evaporated under reduced pressure to afford the expected bromide (4-bromo-2-butynyl)trimethylsilane (1.4 g, 97%) which is used without purification.

EXAMPLE 9

Preparation of (4-BROMO-2-(E)BUTENYL)TRIMETHYLSILANE

4-(Trimethylsilyl)-2(E)-butene-1-ol [H. Mastalerz, J. Org. Chem., 49, 4094, (1984)] (10 g, 70 mmol) is dissolved in dry diethyl ether (150 ml) and phosphorous tribromide (2.2 ml, 23.3 mmol) is added dropwise. Then the mixture is refluxed, protected from the light during 2.5 hours. The reaction is washed twice with water, once with aqueous sodium bicarbonate and then once with water. The organiclayer is dried over sodium sulfate. The solvent is evaporated under reduced pressure to afford the expected bromide (4-bromo-2-(E)butenyl)-trimethylsilane (1.4 g, 97%) which is used without purification.

EXAMPLE 10

Preparation of DIMETHYL(3-IODOPROPYL)PHENYLSILANE

Dimethyl(3-chloropropyl)phenylsilane [J.W. Wilt, W.K. Chwang, C.F. Dockus and N.M. Tomiuk, J. Am. Chem. Soc., 100, 5534, (1978)] (9 g, 48.8 mmol) and sodium iodide (29.5 g, 195 mmol) are refluxed in acetone during 24 hours. The mixture is filtered and the solvent is evaporated under reduced pressure. The residue is dissolved in diethyl ether and washed with water. The organic layer is dried with sodium sulfate, filtered and concentrated under reduced pressure to afford pure dimethyl(3-iodopropyl)phenylsilane as a slightly yellow oil (12.5 g, 93%).

EXAMPLE 11

Preparation of BENZYL(IODOMETHYL)DIMETHYLSILANE

Benzyl(bromomethyl)dimethylsilane [Colm, Earborn and Foad M.S., Malmond J. Organomet. Chem., 209, 13 (1981)] (12 g, 0.05 mmol) and sodium iodide (45 g, 0.3 mmol) are refluxed with stirring in acetone (500 ml) during 24 hours. The reaction mixture is cooled, filtered and the solvent is evaporated under reduced pressure. The residue is dissolved in ether and washed with water. The organic layer is dried over sodium sulfate, filtered and concentrated under reduced pressure to afford benzyl(iodomethyl)-dimethylsilane (13.7 g, 95%) as a slightly yellow oil.

EXAMPLE 12

Preparation of
t-BUTYL(IODOMETHYL)DIMETHYLSILANE

t-Butyl(chloromethyl)dimethylsilane [Makoto Kumada, Mitsuo Ishikawa, Sajiro Meada and Katsuyata Ikura, J. Organometal. Chem. 2, 146, (1964)] (16.4 g, 0.1 mmol) and sodium iodide (60 g, 0.4 mmol) in acetone (500 ml) are refluxed with stirring during 24 hours. The reaction mixture is cooled, filtered and the solvent is evaporated under reduced pressure. The residue is dissolved in ether and washed with water. The organic layer is dried over sodium sulfate, filtered and concentrated under reduced pressure to afford t-butyl(iodomethyl)dimethylsilane (20.9 g, 80%) as a slightly yellow oil.

EXAMPLE 13

Preparation of
5-AZIDO-3,6-DI-O-BENZYL-5-DEOXY-D-GLUCOFURANOSE

The azide 5-azido-3,6-di-O-benzyl-5-deoxy-1,2-O-iso-propylidene-α-D-glucofuranoside (U.G. Nayak and R.L. Whisler, J. Org. Chem., 33, 3582 (1968) (15.02 g, 35.3 mmol) was dissolved at 0 °C in 100 ml of a 9:1 mixture of trifluoro-acetic acid and water. The mixture was stirred at 0 °C during 2 h. The trifluoroacetic acid was evaporated under reduced pressure at room temperature. The residue was taken with ether and washed with water. The organic layer was dried over sodium sulfate, filtered and concentrated under reduced pressure. Flash chromatography on silica gel and elution with a 1:1 mixture of hexane and ethyl acetate, followed by recrystallization in a mixture of hexane and ethyl acetate afforded the expected compound 5-azido-3,6-di-O-benzyl-5-deoxy-D-glucofuranose.

EXAMPLE 14

Preparation of
METHYL 5-AZIDO-3,6-DI-O-BENZYL-5-DEOXY-D-GLUCOFURANOSIDE

To a solution of 5-azido-3,6-di-O-benzyl-5-D-glucofuranose (10.23 g, 26.5 mmol) in methylene chloride (170 ml) was added methanol (11 ml) and borontrifluoroetherate (1.5 ml). The mixture was stirred 24 h at room temperature. The reaction mixture was successively washed with a saturated aqueous solution of sodium bicarbonate and then with brine. The organic layer was dried over sodium sulfate, filtered and concentrated under reduced pressure. Flash chromatography on silica gel and elution with a 1:1 mixture of hexane and ethyl acetate afforded methyl 5-azido-3,6-di-O-benzyl-5-deoxy-D-glucofuranoside as a colorless oil (9.15 g, 85%).

EXAMPLE 15

Preparation of
METHYL 5-AZIDO-2,3,6-TRI-O-BENZYL-5-DEOXY-D-GLUCOFURANOSIDE

To a suspension of sodium hydride (1.2 g, 27.5 mmol), 55% in mineral oil, washed three times with pentane) inanhydrous tetrahydrofuran (200 ml) was added quickly dropwise the alcohol methyl 5-azido-3,6-di-O-benzyl-5-deoxy-D-gluco-furanoside (9.15 g, 22.9 mmol) in tetrahydrofuran (50 ml) at room temperature and under nitrogen. The mixture was stirred during 3 h at room temperature. the mixture was yellow. Then $n\text{-}Bu_4N^+I^-$ (76 mg, 0.20 mmol) was added followed by benzyl bromide (3.30 ml, 27.5 mmol) added dropwise. The mixture was stirred overnight at room temperature. After hydrolysis with saturated aqueous ammonium chloride tetrahydrofuran was evaporated under reduced pressure. The residue was diluted with water and extracted three times with ether. The organic phase was dried over sodium sulfate. Filtration and evaporation under reduced pressure afforded an oil. Flash chromatography on silica gel and elution with a 20:80 mixture of ethyl acetate and hexane afforded the expected compound methyl 5-azido-2,3,6-tri-O-benzyl-5-deoxy-D-glucofuranoside as a colorless oil (10.88 g, 97%).

EXAMPLE 16

Preparation of
5-AZIDO-2,3,6-TRI-O-BENZYL-5-DEOXY-D-GLUCOFURANOSE

Methyl 5-azido-2,3,6-tri-O-benzyl-5-deoxy-D-glucofuranoside (10.8 g, 22.2 mmol) was dissolved at room temperature in tetrahydrofuran (20 ml). The solution was cooled at -10 °C and trifluoroacetic acid (120 ml) was added dropwise followed by addition of water (20 ml). The mixture was stirred at 0 °C during 24 h. The mixture was evaporated under reduced pressure without heating. The residue was taken with ether and washed with water. The organic layer was dried over sodium sulfate, filtered and concentrated under reduced pressure. Flash chromatography on silica gel and elution with a 20:80 mixture of ethyl acetate and hexane afforded 5-azido-2,3,6-tri-O-benzyl-5-deoxy-D-glucofuranose as a colorless oil (9.63 g, 90%).

EXAMPLE 17

Preparation of
5-AZIDO-2,3,6-TRI-O-BENZYL-5-DEOXY-D-GLUCONIC ACID-γ-LACTONE

To a solution of the lactol 5-azido-2,3,6-tri-O-benzyl-5-deoxy-D-glucofuranose (9.36 g, 20 mmol) in acetone (240 ml) cooled to 0 °C, Jones' reagent 2 M (11.5 ml) was added dropwise until the color was orange. The excess of Jones' reagent was destroyed with 2-propanol (0.5 ml). The mixture was concentrated under reduced pressure. The residue was taken with water and extracted with ether. The organic phase was dried with sodium sulfate, filtered and concentrated under reduced pressure so as to afford an oil. Flash chromatography on silica gel and elution with a 1:9 mixture of ethyl acetate and hexane afforded the γ-lactone 5-azido-2,3,6-tri-O-benzyl-5-deoxy-D-gluconic acid-γ-lactone.

EXAMPLE 18

Preparation of
2,3,6-TRI-O-BENZYL-5-DEOXY-D-GLUCONIC ACID-δ-LACTAM

To a solution of the lactone 5-azido-2,3,6-tri-O-benzyl-5-deoxy-D-gluconic acid-γ-lactone (8.16 g, 17 mmol) in ethanol (180 ml) was added Lindlar catalyst (1.7 g). The mixture was hydrogenated under atomospheric pressure during 24 h. Filtration and evaporation under reduced pressure afforded an oil which was crystallized in a mixture of hexane and ether. The lactam 2,3,6-tri-O-benzyl-5-deoxy-D-gluconic acid-δ-lactam was obtained as white crystals (7.4 g, 96%). mp: 85-85.5 °C.

EXAMPLE 19

Preparation of
2,3,6-TRI-O-BENZYL-1,5-DIDEOXY-1,5-IMINO-D-GLUCITOL

To a solution of 2,3,6-tri-O-benzyl-5-deoxy-D-gluconic acid δ-lactam (compound described in Example 18) (0.75 g, 1.6 mmol) in dry tetrahydrofuran (15ml) was added a 10 M solution of borane in methyl sulfide (0.58 ml) under nitrogen at 0 °C. The mixture was stirred 15 min at 0 °C, 30 min at room temperature, then refluxed during 6 h and finally stirred overnight at room temperature. The mixture was cooled to 0 °C and the excess of borane was destroyed with methanol and stirred 1 h at room temperature. The reaction mixture was treated with gazeous hydrochloric acid and refluxed during 1 h. The solvents were evaporated under reduced pressure. The residue was dissolved in ethyl acetate and washed with a saturated aqueous solution of sodium bicarbonate. The organic layer was dried over sodium sulfate, filtered and concentrated under reduced pressure to afford an oil. Flash chromatography on silica gel and elution with ethyl acetate afforded 2,3,6-tri-O-benzyl-1,5-dideoxy-1,5-imino-D-glucitol which crystallized in methanol (0.655 g, 90%); m.p. 73-74 °C.

EXAMPLE 20

Preparation of
1,5-DIDEOXY-1,5-{[4-(TRIMETHYLSILYL)-2-BUTYNYL]IMINO}-D-GLUCITOL

A solution of 1,5-dideoxy-1,5-imino-D-glucitol (0.5 g, 3.06 mmol) and (4-bromo-2-butynyl)trimethylsilane (0.943 g, 4.6 mmol) is dissolved in dimethylformamide (15 ml) containing water (0.5 ml). Triethylamine (0.85 ml) is added. The mixture is heated at 80 °C during 24 hours. The solvent is evaporated under reduced pressure. Flash chromatography on silica gel and elution with a 8:2 mixture of chloroform and methanol affords 1,5-dideoxy-1,5-{[4-(trimethylsilyl)-2-butynyl]imino}-D-glucitol as an amorphous solid (0.24 g, 27%).

EXAMPLE 21

Preparation of
1,5-DIDEOXY-1,5-{[4-(TRIMETHYLSILYL)-2(Z)-BUTENYL])IMINO}-D-GLUCITOL

1,5-Dideoxy-1,5-{[4-(trimethylsilyl)-2-butynyl]imino}-D-glucitol (0.1 g, 0.35 mmol) is dissolved in methanol (5 ml) and Lindlar catalyst (25 mg) is added. The mixture is hydrogenated at atmospheric pressure overnight. The catalyst is filtered off and the solvent is evaporated under reduced pressure to afford 1,5-dideoxy-1,5-{[4-(trimethylsilyl)-2(Z)-butenyl]imino}-D-glucitol as an amorphous solid (0.09 g, 90%).

EXAMPLE 22

Preparation of
1,5-DIDEOXY-1,5-{[4-(TRIMETHYLSILYL)-2(E)-BUTENYL]IMINO}-D-GLUCITOL

A solution of 1,5-dideoxy-1,5-imino-D-glucitol (also known as 1-deoxynojirimycin) (0.5 g, 3.06 mmol) and (4-bromo-2-(E)butenyl)trimethylsilane (0.95 g, 4.6 mmol) in a mixture of dimethylformamide (10 ml), water (0.5 ml) and triethylamine (0.85 ml) is heated at 80 °C during 24 hours. The solvents are evaporated under reduced pressure. Flash chromatography on silica gel and elution with a 8:2 mixture of chloroform and methanol affords 1,5-dideoxy-1,5-{[4-(trimethylsilyl)-2(E)-butenyl]-imino}-D-glucitol as a foam (0.12 g, 14%).

EXAMPLE 23

Preparation of
1,5-DIDEOXY-2,3,6-TRI-O-BENZYL-1,5-{[3-(DIMETHYLPHENYLSILYL)-PROPYL]IMINO}-D-GLUCITOL

A solution of 1,5-dideoxy-2,3,6-tri-O-benzyl-1,5-imino}-D-glucitol(0.433 g, 1 mmol) and dimethyl(3-iodopropyl)-phenylsilane (0.912 g, 3 mmol) in a mixture of dimethyl-formamide (6 ml) and triethylamine (0.42 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-o-benzyl-1,5-{[3-(dimethylphenylsilyl)-propyl]imino}-D-glucitol as a colorless oil (0.493 g, 81%).

EXAMPLE 24

Preparation of
1,5-DIDEOXY-1,5-{[3-(DIMETHYLPHENYLSILYL)PROPYL]IMINO}-D-GLUCITOL

1,5-Dideoxy-2,3,6-tri-O-benzyl-1,5-{[3-(dimethylphenylsilyl)propyl]imino}-D-glucitol (0.45 g, 0.74 mmol) is dissolved in a 9:1 mixture of methanol and formic acid (10 ml), and palladium 10% on charcoal (0.45 g) is added. The mixture is stirred overnight at room temperature. The catalyst is removed by filtration. The solvents are evaporated under reduced pressure. The residue is dissolved in water and passed through a column of Amberlyst A26 $OH^{\ominus}$.Water is evaporated under reduced pressure and flash chromatography on silica gel and elution with a 8:2 mixture of chloroform and methanol affords the expected product 1,5-dideoxy-1,5-{[3-(dimethylphenylsilyl)-propyl]imino}-D-glucitol as an amorphous solid (0.208 g, 83%).

EXAMPLE 25

Preparation of
1,5-DIDEOXY-2,3,6-TRI-O-BENZYL-1,5-{[(BENZYLDIMETHYLSILYL)-METHYL]IMINO}-D-GLUCITOL

A solution of 1,5-dideoxy-2,3,6-tri-O-benzyl-1,5-imino-D-glucitol (0.433 g, 1 mmol) and benzyl-(iodomethyl)dimethylsilane (0.87 g, 3 mmol) in a mixture of dimethylformamide (6 ml) and triethylamine (0.42 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[(benzyldimethylsilyl)methyl]imino}-D-glucitol as a colorless oil (0.386 g, 65%).

EXAMPLE 26

Preparation of
1,5-DIDEOXY-1,5-{[(BENZYLDIMETHYLSILYL)METHYL]IMINO}-D-GLUCITOL

1,5-Dideoxy-2,3,6-tri-O-benzyl-1,5-{[(benzyldimethylsilyl)methyl]imino}-D-glucitol (0.3 g, 0.5 mmol) is dissolved in a 9:1 mixture of methanol and formic acid (10 ml), and palladium 10% on charcoal (0.3 g) is added. The mixture is stirred overnight at room temperature. The catalyst is removed by filtration. The solvents are evaporated under reduced pressure. The residue is dissolved in water and passed through a column of Amberlyst A26 $OH^{\ominus}$.Water is evaporated under reduced pressure and flash chromatography on silica gel and elution with a 8:2 mixture of chloroform and methanol affords the expected product 1,5-dideoxy-1,5-{[(benzyldimethylsilyl)-methyl]-imino}-D-glucitol as an amorphous solid (0.09 g, 55%).

EXAMPLE 27

Preparation of
1,5-DIDEOXY-2,3,6-TRI-O-BENZYL-1,5-{[(t-BUTYLDIMETHYLSILYL)-METHYL]IMINO}-D-GLUCITOL

A solution of 1,5-dideoxy-2,3,6-tri-O-benzyl-1,5-imino-D-glucitol (0.433g, 1 mmol) and t-butyl-(iodomethyl)dimethylsilane (0.77 g, 3 mmol) in a mixture of dimethylformamide (6 ml) and triethylamine (0.42 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[(t-butyldimethylsilyl)-methyl]imino}-D-glucitol as a colorless oil (0.42 g, 75%).

EXAMPLE 28

Preparation of
1,5-DIDEOXY-1,5-{[(t-BUTYLDIMETHYLSILYL)METHYL]IMINO}-D-GLUCITOL

1,5-Dideoxy-2,3,6-tri-O-benzyl-1,5-{[(t-butyldimethylsilyl)methyl]imino}-D-glucitol (0.4 g, 0.72 mmol) is dissolved in a 9:1 mixture of methanol and formic acid (10 ml), and palladium 10% on charcoal (0.5 g) is added. The mixture is stirred overnight at room temperature. The catalyst is removed by filtration. The solvents are evaporated under reduced pressure. The residue is dissolved in water and passed through a column of Amberlyst A26 $OH^{\ominus}$.Water is evaporated under reduced pressure and flash chromatography on silica gel and elution with a 8:2 mixture of chloroform and methanol affords the expected product 1,5-dideoxy-1,5-{[(t-butyldimethylsilyl)-methyl]imino}-D-glucitol as an amorphous solid (0.127 g, 61%).

EXAMPLE 29

Preparation of
1,5-DIDEOXY-2,3,6-TRI-O-BENZYL-1,5-{[(DIMETHYLPHENYLSILYL)-METHYL]IMINO}-D-GLUCITOL

A solution of 1,5-dideoxy-2,3,6-tri-O-benzyl-1,5-imino-D-glucitol (0.433 g, 1 mmol) and phenyl-(iodomethyl)dimethylsilane [Chih-Tang Huang and Pao-Jen Wang, Hua Hsüeh Hsüeh Pao, 25, 341, (1959)] (0.77 g, 3 mmol) in a mixture of dimethylformamide (6 ml) and triethylamine (0.42 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[-(dimethylphenylsilyl)-methyl]imino}-D-glucitol as a colorless oil (0.37 g, 64%).

EXAMPLE 30

Preparation of
1,5-DIDEOXY-1,5-{[(DIMETHYLPHENYLSILYL)METHYL]IMINO}-D-GLUCITOL

1,5-Dideoxy-2,3,6-tri-O-benzyl-1,5-{[(dimethylphenylsilyl)methyl]imino}-D-glucitol (0.35 g, 0.60 mmol) is dissolved in a 9:1 mixture of methanol and formic acid (10 ml), and palladium 10% on charcoal (0.35 g) is added. The mixture is stirred overnight at room temperature. The catalyst is removed by filtration. The solvents are evaporated under reduced pressure. The residue is dissolved in water and passed through a column of Amberlyst A26 $OH^{\ominus}$.Water is evaporated under reduced pressure and flash chromatography on silica gel and elution with a 8:2 mixture of chloroform and methanol affords the expected product 1,5-dideoxy-1,5-{[(dimethylphenylsilyl)-methyl]imino}-D-glucitol as an amorphous solid (0.139 g, 75%).

EXAMPLE 31

Preparation of
1,5-DIDEOXY-2,3,6-TRI-O-BENZYL-1,5-{[(TRIMETHYLSILYL)-METHYL]IMINO}-D-GLUCITOL

A solution of 1,5-dideoxy-2,3,6-tri-O-benzyl-1,5-imino-D-glucitol (0.1 g, 0.24 mmol) and (iodomethyl)-trimethylsilane (0.45 ml, 3.1 mmol) in a mixture of dimethylformamide (3 ml) and triethylamine (0.44 ml) is heated at 80 °C during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[-(trimethylsilyl)methyl]imino}-D-glucitol as a colorless oil (0.09 g, 75%).

EXAMPLE 32

Preparation of
1,5-DIDEOXY-1,5-{[(TRIMETHYLSILYL)METHYL]IMINO}-D-GLUCITOL

1,5-Dideoxy-2,3,6-tri-O-benzyl-1,5-{[(trimethylsilyl)-methyl]imino}-D-glucitol (0.075 g, 0.14 mmol) is dissolved in a 9:1 mixture of methanol and formic acid (12 ml), and palladium 10% on charcoal (0.3 g) is added. The mixture is stirred overnight at room temperature. The catalyst is removed by filtration. The solvents are evaporated under reduced pressure. The residue is dissolved in water and neutralized with AGI-X8, 20-50 mesh, $OH^{\ominus}$ form. The resin is removed by filtration, water is removed by lyophilization and the expected product 1,5-dideoxy-1,5-{[(trimethylsilyl)-methyl]imino}-D-glucitol is obtained as an amorphous solid (0.016 g, 45%).

EXAMPLE 33

Preparation of
1,5-DIDEOXY-2,3,6-TRI-O-BENZYL-1,5-{[3-(TRIMETHYLSILYL)-PROPYL]IMINO}-D-GLUCITOL

A solution of 1,5-dideoxy-2,3,6-tri-O-benzyl-1,5-imino-D-glucitol (0.34 g, 0.78 mmol) and (3-iodopropyl)-trimethylsilane (0.57 gr, 2.34 mmol) in a mixture of dimethylformamide (5 ml) and triethylamine (0.33 ml) is stirred at room temperature during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[3-(trimethylsilyl)-propyl]imino}-D-glucitol as a colorless oil (0.405 g, 94%).

EXAMPLE 34

Preparation of
1,5-DIDEOXY-1,5-{[3-(TRIMETHYLSILYL)PROPYL]IMINO}-D-GLUCITOL

1,5-Dideoxy-2,3,6-tri-O-benzyl-1,5-{[3-(trimethylsilyl)-propyl]imino}-D-glucitol (0.39 g, 0.7 mmol) is dissolved in a 9:1 mixture of methanol and formic acid (30 ml), and palladium 10% on charcoal (2 g) is added. The mixture is stirred overnight at room temperature. The catalyst is removed by filtration. The solvents are evaporated under reduced pressure. The residue is dissolved in water and neutralized with AGI-X8, 20-50 mesh, $OH^{\ominus}$ form. The resin is removed by filtration, water is removed by lyophilization and the expected product 1,5-dideoxyl-5-{[3-(trimethylsilyl)-propyl]imino}-D-glucitol is obtained as an amorphous solid (0.17 g, 85%).

EXAMPLE 35

Preparation of
1,5-DIDEOXY-2,3,6-TRI-O-BENZYL-1,5-{[4-(TRIMETHYLSILYL)-BUTYL]-IMINO}-D-GLUCITOL

A solution of 1,5-dideoxy-2,3,6-tri-O-benzyl-1,5-imino-D-glucitol (0.043 g, 0.1 mmol) and (4-iodobutyl)-trimethylsilane (0.088 g, 0.3 mmol) in a mixture of dimethylformamide (0.8 ml) and triethylamine (0.04 ml) is stirred at room tenperature during 24 hours. The solvents are evaporated under reduced pressure. The residue is dissolved in ethyl acetate, washed with water. The organic layer is dried over sodium sulfate, filtered and concentrated under reduced pressure to afford an oil. Flash chromatography on silica gel and elution with a 8:2 mixture of hexane and ethyl acetate affords the expected product 1,5-dideoxy-2,3,6-tri-O-benzyl-1,5-{[4-(trimethylsilyl)butyl]imino}-D-glucitol as a colorless oil (0.05 g, 90%).

EXAMPLE 36

Preparation of
1,5-DIDEOXY-1,5-{[4-(TRIMETHYLSILYL)BUTYL]IMINO}-D-GLUCITOL

1,5-Dideoxy-2,3,6-tri-O-benzyl-1,5-{[4-(trimethylsilyl)butyl}imino}-D-glucitol (0.05 g, 0.09 mmol) is dissolved in a 9:1 mixture of methanol and formic acid (15 ml), and palladium 10% on charcoal (0.2 g) is added. The mixture is stirred overnight at room temperature. The catalyst is removed by filtration. The solvents are evaporated under reduced pressure. The residue is dissolved in water and neutralized with AGI-X8, 20-50 mesh, $OH^{\ominus}$ form. The resin is removed by filtration, water is removed by lyophilization and the expected product 1,5-dideoxy-1,5-{[4-(trimethylsilyl)butyl]imino}-D-glucitol is obtained as an amorphous solid (0.02 g, 75%).

EXAMPLE 37

Preparation of
1,5-DIDEOXY-1,5-IMINO-D-GLUCITOL: DEOXYNOJIRIMYCIN

2,3,6-Tri-O-benzyl-1,5-dideoxy-1,5-imino-D-glucitol (0.2 g, 0.46 mmol) is dissolved in a 9:1 mixture of methanol and formic acid (10 ml) under an inert atmosphere and palladium 10% on charcoal (0.4 g) is added. The mixture is stirred overnight at room temperature. The catalyst is removed by filtration. The solvents are evaporated under reduced pressure. The residue is dissolved in methanol and the solution is filtered through a membrane (Millex-SR 0.5μM). Evaporation of solvent gives a sticky solid which is further triturated in ethanol to give the expected 1,5-dideoxy-1,5-imino-D-glucitol as a beige powder (60 mg, 80%).

EXAMPLE 38

Preparation of
(E)-3-TRIMETHYLSILYL-2-PROPEN-1-OL

2 g (15.6 mmol) of 1-trimethylsilyl-2-propyn-1-ol in 10 ml of dry ether are added dropwise to an ice-cooled solution of sodium bis(2-methoxyethoxy) aluminum [Red-Al. 3.4 M in toluene, (7.3 ml, 25.1 mmol)] in 10 ml of dry ether. The reaction mixture is then further stirred during 2½ hours at room temperature and poured into an ice-cooled sulfuric acid (1 N) ether mixture. If necessary the pH is adjusted to be slightly basic, the organic phase is then removed and the aqueous phase is further extracted with ether. The combined organic phases are dried over sodium sulfate, filtered and concentrated under reduced pressure. Rapid flash chromatography of the residue on a silica gel column eluted with a 8:2 mixture of hexane and ethyl acetate affords the expected (E)-3-trimethylsilyl-2-propen-1-ol as a colorless liquid (1.8 g, 90%).

EXAMPLE 39

Preparation of
(E)-3-TRIMETHYLSOLYL-2-PROPEN-1-OL, METHANESULFONATE

1.6 ml (11.5 mmol) of triethylamine and 0.74 ml (9.6 mmol) of methanesulfonylchloride in 10 ml of dry dichloromethane are successively added to an ice-cooled solution of 1 g (7.6 mmol) of (E)-3-trimethylsilyl-2-propen-1-ol in 20 ml of dry dichloromethane. The reaction mixture is then further stirred during 3 hours at room temperature and poured into a water-dichloromethane mixture, the organic phase is removed, the aqueous phase is further extracted with dichloromethane. The organic phases are combined and washed with sodium bicarbonate, dried over sodium sulfate, filtered and concentrated under reduced pressure to give 1.1 g of a yellowish liquid which is further bulb to bulb distilled under reduced pressure (water pump, oven temperature 150-200 °C) to afford 0.8 g (50%) of the expected (E)-3-trimethylsilyl-2-propen-1-ol, methanesulfonate.

EXAMPLE 40

Preparation of
1,5-DIDEOXY-1,5-{[3-(TRIMETHYLSILYL)-2-PROPENYL)IMINO}-D-GLUCITOL

A solution of 1,5-dideoxy-1,5-imino-D-glucitol (0.052 g, 0.32 mmol) and (E)-3-trimethylsilyl-2-propen-1-ol, methane-sulfonate (0.133 g, 0.63 mmol) in a mixture of dimethyl-formamide (2 ml) and triethylamine (0.09 ml, 0.63 mmol) is heated at 80 °C during 20 hours. Solvents are evaporated under reduced pressure and the residue is flash chromatographed on a silica gel column, eluted with dichloromethane: ethanol 9:1 to 7:3 to give 0.022 g (35%) of the expected 1,5-dideoxy-1,5-{[3-(trimethylsilyl)-2-propenyl]imino}-D-glucitol as a white powder.

EXAMPLE 41

Preparation of
1-METHYL-3-TRIMETHYLSILYL-BENZENE

A mixture of 9.2 g (50 mmol) of 3-bromo-toluene and 5.84 g (50 mmol) of chlorotrimethylsilane in 100 ml of dry ether are added dropwise to 1.2 g (50 mmol) of magnesium turnings in a few milliliters of ether, some crystals of iodine are added to start the reaction. The reaction mixture is refluxed for 20 hours after the end of the addition and then poured into a saturated solution of ammonium chloride (200 ml). The aqueous solution is further extracted with ether, the ethereal extracts are combined, dried over sodium sulfate, filtered and concentrated under reduced pressure to afford 4.4 g of a yellowish liquid. Flash chromatography on a silica gel column and elution with petroleum ether give 2.3 g (30%) of the expected 1-methyl-3-trimethylsilyl-benzene as a colorless liquid.

EXAMPLE 42

Preparation of
1-BROMOMETHYL-3-TRIMETHYLSILYL-BENZENE

A mixture of 2.5 g (15 mmol) of 1-methyl-3-trimethylsilyl-benzene and 3.1 g (16.5 mmol) of N-bromosuccinimide in 80 ml of carbon tetrachloride are refluxed in the presence of a catalytic amount of benzoyl peroxide. When succinimide has totally precipitated at the surface, the hot mixture is filtered. The filtrate is evaporated under reduced pressure, the residue obtained is dissolved in a brine-chloroform mixture. The organic phase is separated, dried over sodium sulfate, filtered and evaporated to afford 4 g of a yellowish liquid. Flash chromatography on silica gel and elution with petroleum ether give 2g (60%) of the expected 1-bromomethyl-3-trimethylsilyl-benzene as a colorless liquid.

EXAMPLE 43

Preparation of
1,5-DIDEOXY-1,5-[{[3-(TRIMETHYLSILYL)PHENYL]METHYL}IMZNO]-D-GLUCITOL

A solution of 1,5-dideoxy-1,5-imino-D-glucitol (0.128 g, 0.78 mmol) and 1-bromomethyl-3-trimethylsilyl-benzene (0.3 g, 1.23 mmol) in a mixture of dimethylformamide (5 ml) and triethylamine (0.17 ml) is heated at 100 °C during 20 hours. Solvents are evaporated under reduced pressure and the residue is flash chromatographed on a silica gel column, eluted with dichloromethane:ethanol 9:1 to 7:3 to give 0.1 g (40%) of the expected 1,5-dideoxy-1,5-[{[3-(trimethylsilyl)-phenyl]methyl}imino]-D-glucitol as a white powder.

EXAMPLE 44

Preparation of
1,5-DIDEOXY-1,5-{[3-(TRIMETHYLSILYL)PROPYL]IMINO}-D-GLUCITOL

Step A:

3-Iodopropyltrimethylsilane

A stirred solution of 3-chloropropyltrimethylsilane (5.0 g, 33 mmol) and NaI (7.5 g, 50 mmol) in acetone (45 ml) was heated at a reflux for 16 hours. The solution became dark yellow (iodine) with a white precipitate (NaCl). The resultant mixture was cooled to room temperature and filtered to remove the NaCl. The salt was washed with acetone (3 × 5 ml). The washings were combined with the filtrate and concentrated (25 °C/10 Torr) leaving a two-phase mixture. The concentrate was partitioned in ethyl acetate (50 ml) and water (25 ml). The organic layer was separated, washed with aqueous sodium metabisulfite 10% (10 ml), water (20 ml), dried ($MgSO_4$), and concentrated (25 °C/10 Torr) to give 6.0 g of a pale yellow oil. This crude oil was distilled through a six-inch Vigreux column (removing some unreacted chloropropyltrimethylsilane) under water aspirator vacuum giving 4.2 g (50%) of a colorless oil: b.p. 72 °C/10 Torr.

Step B:

1,5-Dideoxy-1,5-{[3-(trimethylsilyl)propyl]imino}-D-glucitol

A well stirred mixture of 1,5-dideoxy-1,5-imino-D-glucitol (0.50 g, 3.1 mmol), 3-iodopropyltrimethylsilane (1.1 g, 4.6 mmol) and sodium bicarbonate (0.38 g, 4.6 mmol) in sulfolane (5 ml) was heated at 90 °C for 3 hours and then cooled to room temperature. The mixture was diluted with water (5 ml), acidified with 1 M HCl (5 ml) to pH 2-3, and allowed to stir at room temperature for 1 hour. The mixture was washed with hexane (3 × 5 ml) and adjusted to pH 8 with 1 M sodium hydroxide (3.2 ml) precipitating the crude product. The mixture was cooled to 5 °C (ice-water bath), filtered, and the filter cake was washed with ice-water (2 × 3 ml) and air-dried for 1 hour to give 0.64 g of a white solid. The solid was dissolved in hot (80 °C) water (12 ml) and cooled to give 0.56 g (66%) of the desired product as white plates.

Enzymes which catalyze the hydrolysis of complex carbohydrates, e.g. α-glycosidases, convert non-absorbable carbohydrates into absorbable sugars. The rapid action of these enzymes, particularly following the intake of high levels of carbohydrates, lead to acute high levels in blood glucose which, in the case diabetics, lead to undesirable manifestations, thus it has been a long-sought goal to find compounds which will obviate the hyperglycemia caused by dietary improprieties. Similarly, in the case of obesity the control of high levels of blood glucose, with its subsequent conversion to fat, caused by the catalysis of carbohydrates has inspired the quest for compounds which will obviate the problems associated with dietary improprieties. "Dietary improprieties" means eating habits associated with overeating, overdrinking, and failure to maintain a balanced diet such as the excessive intake of carbohydrates, which metabolize to glucose and which lead to obesity.

The compound of the present invention are administered to subjects in need of such therapy, e.g., mammals such as humans. The compounds of this invention (I) are potent and long-lasting inhibitors of α-glucosidase and, by standard laboratory methods for determining serum glucose levels, are shown to be useful for the treatment of disease states caused by the underutilization and/or overproduction of serum glucose without adversely affecting the rate of transport across cell membranes. Thus, the compounds are useful in the treatment of diabetes and obesity.

In the practice of this invention, an effective amount of a compound of this invention is that amount required to reduce the amount of serum glucose (relative to a control) following the ingestion of carbohydrates convertible to absorbable glucose. The specific dosage for the treatment of any specific patient suffering from either disease state will depend upon such factors as size, type and age of the patient as well as the patients' dietary habits and the severity of the disease state, all of which are factors normally familiar to and considered by the attending diagnostician treating the patient. Generally, the compounds are to be administered orally at a dose of 0.01 to 2 milligrams per kilogram of body weight (MPK) with a dose of 0.025 to 0.5 MPK being preferred. The compounds preferably are to be administered orally at mealtimes in single or multiple unit doses containing 1 mg to 10 mg. Of course, in the treatment of obesity, the term includes the practice of treating the disease as well as continued administration of dose regimens suitable for the maintenance of the desired weight for the patient.

It is also to be found that the compounds of the instant invention (I) will exert an inhibitory effect on glycosidase enzymes that are essential for elaboration of the final structure of the oligosaccharide side-chains of glycoproteins, particularly the HIV (gp 120) glycoprotein. Suitable assay techniques, e.g. syncytial formation, the reverse transcriptase assay, immunofluorescence tests and election microscopy, may be used to evaluate the effects on HIV viral growth and for determining dose regimens. Anti-viral effects may be confirmed by immunofluorescence with serum for virally infected patients. In the treatment of the HIV related disease states (e.g., AIDS), as well as other retroviral glyco-protein-related disease states, unlike the treatment of diabetes and obesity, the compounds of this invention may be administered by parenteral means; specific doses being within the above stated dose range for treatment of diabetes and obesity. In addition to treating AIDS with the compounds of this invention, the compounds of this invention may also be effectively utilized in conjunctive therapy with compounds known to be useful in treating patients with AIDS such as for example 2,3-dideoxycytidine, 2,3-dideoxyadenine, interferon, interleukin-2 and the like.

In practising the end-use application of the compounds of this invention, the compounds are preferably incorporated in a pharmaceutical formulation comprising a pharmaceutical carrier in admixture with a compound of this invention. The term "pharmaceutical carrier" refers to known pharmaceutical excipients useful in formulating pharmaceutically active compounds for internal administration to animals, and which are substantially non-toxic and non-sensitizing under conditions of use. The compositions can be prepared by known techniques for the preparation of tablets, capsules, elixirs, syrups emulsions, dispersions and wettable and effervescent powders, and can contain suitable excipients known to be useful in the

preparation of the particular type of composition desired. Suitable pharmaceutical carriers and formulation techniques are found in standard texts, such as Remington's Pharmaceutical Sciences, Mack Publishing Company, Easton, PA.

As is true for most classes of therapeutic agents certain subgeneric groups and certain specific compounds are preferred. For the compounds embraced within this application the preferred sub-generic groups are those wherein Q is $C_{1-7}$ alkylene, $(CH_2)_mCH=CH(CH_2)_n$, $(CH_2)_p$ phenylene, $(CH_2)_m$ cyclopentenylene, $(CH_2)_m$ cyclohexenylene or $(CH_2)_pT$ moieties. Preferred $R_1$ moieties are $C_{1-7}$ alkyl, phenyl or a hydroxylated alkyl, and preferred $R_2$ and $R_3$ moieties are $C_{1-10}$ alkyl, phenyl or benzyl.

The following compounds of Formula 5 illustrate the preferred specific compounds:

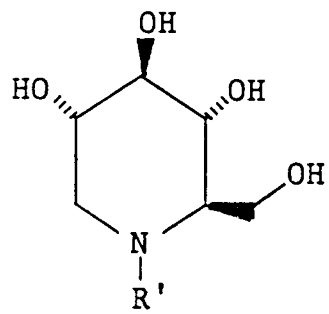

(5)

R'

$-CH_2-Si(CH_3)_3$

$-(CH_2)_3-Si(CH_3)_3$

$-(CH_2)_4-Si(CH_3)_3$

$-CH_2-Si(CH_3)_2C_6H_5$

$-CH_2-Si(CH_3)_2C_2H_5$

$-CH_2-Si(CH_3)_2C_3H_7$

$-CH_2-CH=CH-Si(CH_3)_3$ (trans)

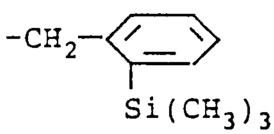

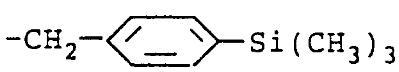

$-(CH_2)_3-Si(CH_3)_2CH_2OH$

$-(CH_2)_3-Si(CH_3)_2CH_2CHOHCH_2OH$

R'

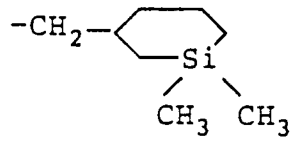

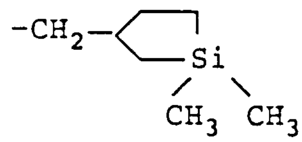

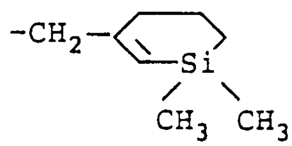

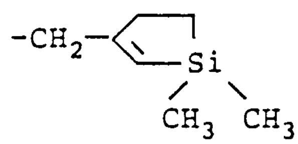

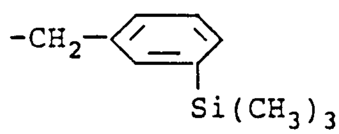

## Claims

**Claims for the following Contracting States : AT, BE, CH, DE, DK, FR, GB, IT, LI, LU, NL, SE**

1. A compound of the formula

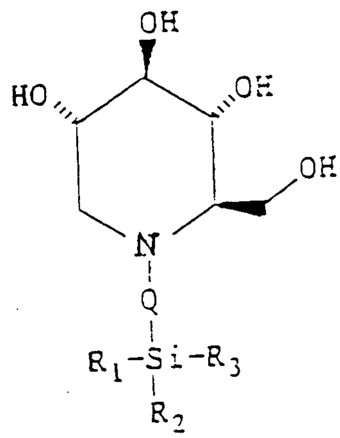

the geometric isomers thereof, and the pharmaceutically acceptable salts thereof wherein

| | |
|---|---|
| Q | is $C_{1-7}$ alkylene, $(CH_2)_mCH=CH(CH_2)_n$, $(CH_2)_mC\equiv C(CH_2)_n$,$(CH_2)_mCH=C=CH(CH2)_n$, $(CH_2)_p$ phenylene, $(CH_2)_m$ cyclopentenylene, $(CH_2)_m$ cyclohexenylene, $(CH_2)_pT$, wherein T, together with the silicon atom to which it is attached forms a 5 to 6 atom cyclic silicane, said cyclic silicane optionally having a double bond, with m being 1,2 or 3, n being 0, 1 or 2, p being 0, 1, 2, 3 or 4, |
| $R_1$ | is $C_{1-7}$ alkyl, $C_{1-7}$ alkoxy, mono-or polyhydroxy $C_{1-6}$ alkyl, mono-or polyalkoxy $C_{1-6}$ alkyl, chloro $C_{1-6}$ alkyl, |
| $R_2$ and $R_3$ | are $C_{1-10}$ alkyl, $(CH_2)_p$-X,Y-Substituted phenyl, with X and Y each being H, OH, halogen, $C_{1-6}$ alkyl, $C_{1-6}$ alkoxy, $CF_3$, CN, $NO_2$, SH or -S-$C_{1-6}$ alkyl, with the proviso that when Q is $(CH_2)_pT$, then one of $R_1$, $R_2$ or $R_3$ is deleted. |

2. The compound of claim 1, wherein Q is a $C_{1-7}$ alkylene.

3. The compound of claim 1, wherein Q is $(CH_2)_mHC=CH(CH_2)_n$.

4. The compound of claim 2 or 3, wherein $R_1$, $R_2$ and $R_3$ are $C_{1-7}$ alkyl.

5. The compound of claim 2, wherein the $C_{1-7}$ alkyl is methyl.

6. The compound of claim 2, wherein $R_1$ is a hydroxylated $C_{1-7}$ alkyl.

7. The compound of claim 1, wherein Q is $(CH_2)_pT$.

8. The compound of claim 7, wherein $R_1$ and $R_2$ are $C_{1-7}$ alkyl and $R_3$ is deleted.

9. The compound of claim 8, wherein the cyclic silicane of $(CH_2)_pT$ is unsaturated.

10. The compound of claim 1, wherein Q is -$CH_2$-phenylene and $R_1$, $R_2$ and $R_3$ are each $C_{1-7}$ alkyl.

11. The compound of claim 1, wherein -Q-Si-$R_1R_2R_3$ is selected from the following groups:
$-CH_2-Si-(CH_3)_3$,
$-(CH_2)_3-Si(CH_3)_3$,
$-(CH_2)_4-Si(CH_3)_3$,
$-CH_2Si(CH_3)_2C_6H_5$,
$-CH_2Si(CH_3)_2C_2H_5$,
$-CH_2Si(CH_3)_2C_3H_7$,

trans-$CH_2$-CH = CH-Si$(CH_3)_3$,
-$CH_2$-$C_6H_4$-meta-Si$(CH_3)_3$,
-$(CH_2)_3$-Si$(CH_3)_2CH_2OH$,
-$(CH_2)_3$-Si$(CH_3)_2CH_2CHOHCH_2OH$,

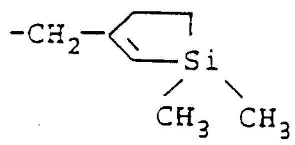

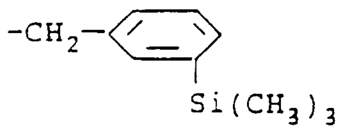

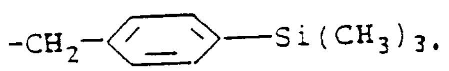

**12.** The compound of claim 1 further comprising a pharmaceutically acceptable carrier.

**13.** A method for preparing compounds of the formula

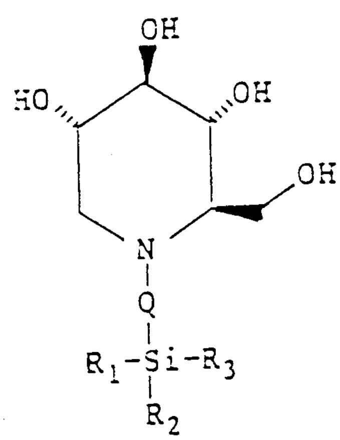

the geometric isomers thereof, and the pharmaceutically acceptable salts thereof wherein

Q is $C_{1-7}$ alkylene, $(CH_2)_mCH=CH(CH_2)_n$, $(CH_2)_mC\equiv C(CH_2)_n$,$(CH_2)_mCH=C=CH(CH_2)_n$, $(CH_2)_p$ phenylene, $(CH_2)_m$ cyclopentenylene, $(CH_2)_m$ cyclohexenylene, $(CH_2)_pT$, wherein T, together with the silicon atom to which it is attached forms a 5 to 6 atom cyclic silicane, said cyclic silicane optionally having a double bond, with m being 1,2 or 3, n being 0,1 or 2, p being 0, 1, 2, 3 or 4,

$R_1$ is $C_{1-7}$ alkyl, $C_{1-7}$ alkoxy, mono-or polyhydroxy $C_{1-6}$ alkyl, mono-or polyalkoxy $C_{1-6}$ alkyl, chloro $C_{1-6}$ alkyl,

$R_2$ and $R_3$ are $C_{1-10}$ alkyl, $(CH_2)_p$-X,Y-substituted phenyl, with X and Y each being H, OH, halogen, $C_{1-6}$ alkyl, $C_{1-6}$ alkoxy, $CF_3$, CN, $NO_2$, SH or -S-$C_{1-6}$ alkyl, with the proviso that when Q is $(CH_2)_pT$, then one of $R_1$, $R_2$ or $R_3$ is deleted which comprises condensing an optionally hydroxy-protected compound of the formula

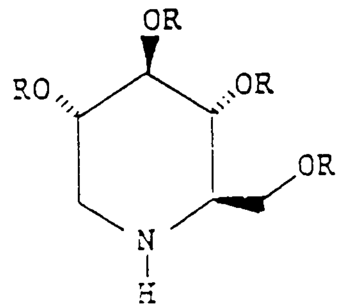

wherein R is H or an optional protecting group with a reactant X'-Q-$SiR_1R_2R_3$ wherein X' is halogeno, mesylate or tosylate followed by the removal of any hydroxy-protecting group and, if necessary, neutralizing any anion formed during the deprotection process.

**14.** The compound according to claim 1, for use as pharmaceutically active agent.

**15.** Use of the compound according to claims 1 to 13 for the preparation of medicaments useful for the treatment of hyperglycemia.

**16.** Use of the compound according to claims 1 to 13 for the preparation of medicaments useful for the treatment of obesity associated with dietary improprieties.

**17.** Use of the compound according to claims 1 to 13 for the preparation of medicaments useful for the treatment of AIDS.

**Claims for the following Contracting State : ES**

**1.** Process for the obtention of a compound of formula:

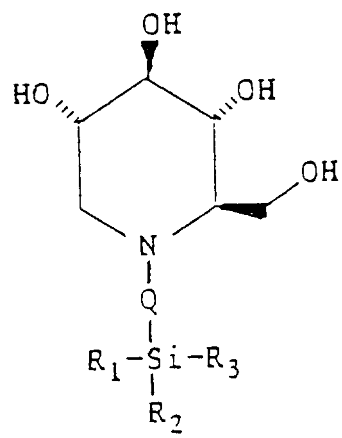

the geometric isomers thereof, and the pharmaceutically acceptable salts thereof wherein

| | |
|---|---|
| Q | is $C_{1-7}$ alkylene, $(CH_2)_mCH=CH(CH_2)_n$, $(CH_2)_mC{\equiv}C(CH_2)n$, $(CH_2)_mCH=C=CH(CH_2)_n$, $(CH_2)_p$ phenylene, $(CH_2)_m$ cyclopentenylene, $(CH_2)_m$ cyclohexenylene, $(CH_2)_pT$, wherein T, together with the silicon atom to which it is attached forms a 5 to 6 atom cyclic silicane, said cyclic silicane optionally having a double bond, with m being 1, 2 or 3, n being 0, 1 or 2, p being 0, 1, 2, 3 or 4, |
| $R_1$ | is $C_{1-7}$ alkyl, $C_{1-7}$ alkoxy, mono-or polyhydroxy $C_{1-6}$ alkyl, mono-or polyalkoxy $C_{1-6}$ alkyl, chloro $C_{1-6}$ alkyl, |
| $R_2$ and $R_3$ | are $C_{1-10}$ alkyl, $(CH_2)_p$-X,Y-substituted phenyl, with X and Y each being H, OH, halogen, $C_{1-6}$ alkyl, $C_{1-6}$ alkoxy, $CF_3$, CN, $NO_2$, SH or -S-$C_{1-6}$ alkyl, with the proviso that when Q is $(CH_2)_pT$, then one of $R_1$, $R_2$ or $R_3$ is deleted which comprises condensing an optionally hydroxy-protected compound of the formula |

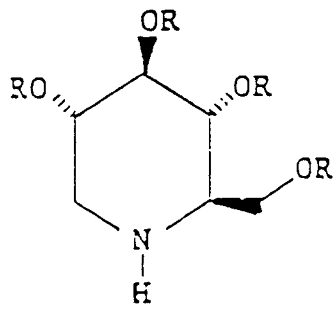

wherein R is H or an optional protecting groupe with a reactant $X'-Q-SiR_1R_2R_3$ wherein X' is halogeno, mesylate or tosylate followed by the removal of any hydroxy-protecting group and, if necessary, neutralizing any anion formed during the deprotection process.

2. Process of claim 1, wherein a reactant $X'-Q-SiR_1R_2R_3$ in which Q is a $C_{1-7}$ alkylene is used to yield a compound of formula (I) in which Q is a $C_{1-7}$ alkylene.

3. Process of claim 1, wherein a reactant $X'-Q-SiR_1R_2R_3$ in which Q is $(CH_2)_mHC=CH(CH_2)_n$ is used to yield a compound of formula I in which Q is $(CH_2)_mHC=CH(CH_2)_n$.

4. Process of claim 1, wherein a reactant $X'-Q-SiR_1R_2R_3$ in which $R_1$, $R_2$ and $R_3$ are $C_{1-7}$ alkyl is used to yield a compound of formula (I) in which $R_1$, $R_2$ and $R_3$ are $C_{1-7}$ alkyl.

5. Process according to claim 4, wherein the $C_{1-7}$ alkyl is methyl.

6. Process of claim 1, wherein a reactant $X'-Q-SiR_1R_2R_3$ in which $R_1$ is a hydroxylated $C_1$-$C_7$ alkyl is used to yield a compound of formula (I) in which $R_1$ is a hydroxylated $C_{1-7}$ alkyl.

7. Process of claim 1, wherein a reactant $X'-Q-SiR_1R_2R_3$ in which Q is $(CH_2)_pT$ is used to yield a compound of formula (I) in which Q is $(CH_2)_pT$

8. Process of claim 1, wherein a reactant $X'-Q-SiR_1R_2R_3$ in which $R_1$ and $R_2$ are $C_{1-7}$ alkyl and $R_3$ is deleted is used to yield a compound of formula (I) in which $R_1$ and $R_2$ are $C_{1-7}$ alkyl and $R_3$ is deleted.

9. Process according to claim 8, wherein a reactant $X'-Q-SiR_1R_2R_3$ in which Q is $(CH_2)_pT$ in which T with the silicon to which it is attached forms an unsaturated cyclic silicane is used to form a compound of formula (I) in which $(CH_2)_pT$ is an unsaturated cyclic silicane.

10. Process of claim 1, wherein a reactant $X'-Q-SiR_1R_2R_3$ in which Q is $-CH_2$-phenylene and $R_1$, $R_2$ and $R_3$ are each $C_{1-7}$ alkyl is used to yield a compound of formula (I) in which Q is $CH_2$-phenylene and $R_1$, $R_2$ and $R_3$ are each $C_{1-7}$ alkyl.

11. Process of claim 1, wherein a reactant $X'-Q-SiR_1R_2R_3$ in which $Q-SiR_1R_2R_3$ is selected from the following groups:
$-CH_2-Si-(CH_3)_3$,
$-(CH_2)_3-Si(CH_3)_3$,
$-(CH_2)_4-Si(CH_3)_3$,
$-CH_2Si(CH_3)_2C_6H_5$,
$-CH_2Si(CH_3)_2C_2H_5$,
$-CH_2Si(CH_3)_2C_3H_7$,
trans-$CH_2-CH=CH-Si(CH_3)_3$,
$-CH_2-C_6H_4$-meta-$Si(CH_3)_3$,
$-(CH_2)_3-Si(CH_3)_2CH_2OH$,
$-(CH_2)_3-Si(CH_3)_2CH_2CHOHCH_2OH$,

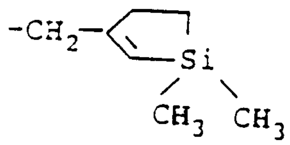

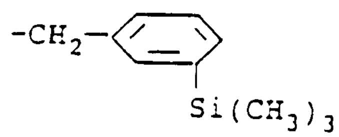

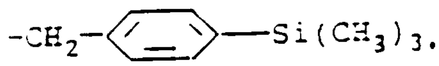

in order to yield a compound of formula (I) in which Q-$SiR_1R_2R_3$ has the above meanings.

**12.** Use of the compound obtained according to the process of anyone of claims 1 to 11 for the preparation of medicaments useful for the treatment of hyperglycemia.

**13.** Use of the compound obtained according to the process of anyone of claims 1 to 11 for the preparation of medicaments useful for the treatment of obesity associated with dietary improprieties.

**14.** Use of the compound obtained according to the process of anyone of claims 1 to 11 for the preparation of medicaments useful for the treatment of AIDS.

**Claims for the following Contracting State : GR**

**1.** A compound of the formula

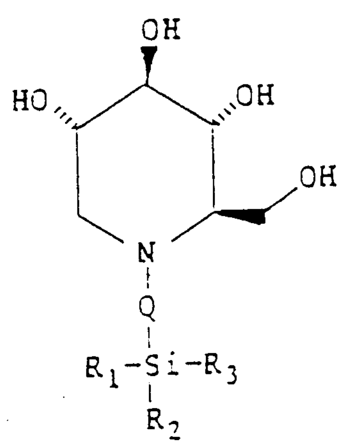

the geometric isomers thereof, and the pharmaceutically acceptable salts thereof wherein

Q is $C_{1-7}$ alkylene, $(CH_2)_mCH=CH(CH_2)_n$, $(CH_2)_mC\equiv C(CH_2)_n$,$(CH_2)_mCH=C=CH(CH_2)_n$, $(CH_2)_p$ phenylene, $(CH_2)_m$ cyclopentenylene, $(CH_2)_m$ cyclohexenylene, $(CH_2)_pT$, wherein T, together with the silicon atom to which it is attached forms a 5 to 6 atom cyclic silicane, said cyclic silicane optionally having a double bond, with m being 1, 2 or 3, n being 0, 1 or 2, p being 0, 1, 2, 3 or 4,

$R_1$ is $C_{1-7}$ alkyl, $C_{1-7}$ alkoxy, mono-or polyhydroxy $C_{1-6}$ alkyl, mono-or polyalkoxy $C_{1-6}$ alkyl, chloro $C_{1-6}$ alkyl,

$R_2$ and $R_3$ are $C_{1-10}$ alkyl, $(CH_2)_p$-X,Y-substituted phenyl, with X and Y each being H, OH,

halogen, $C_{1-6}$ alkyl, $C_{1-6}$ alkoxy, $CF_3$, CN, $NO_2$, SH or -S-$C_{1-6}$ alkyl, with the proviso that when Q is $(CH_2)_pT$, then one of $R_1$, $R_2$ or $R_3$ is deleted.

**2.** The compound of claim 1, wherein Q is a $C_{1-7}$ alkylene.

**3.** The compound of claim 1, wherein Q is $(CH_2)_mHC=CH(CH_2)_n$.

**4.** The compound of claim 2 or 3, wherein $R_1$, $R_2$ and $R_3$ are $C_{1-7}$ alkyl.

**5.** The compound of claim 2, wherein the $C_{1-7}$ alkyl is methyl.

**6.** The compound of claim 2, wherein $R_1$ is a hydroxylated $C_{1-7}$ alkyl.

**7.** The compound of claim 1, wherein Q is $(CH_2)_pT$.

**8.** The compound of claim 7, wherein $R_1$ and $R_2$ are $C_{1-7}$ alkyl and $R_3$ is deleted.

**9.** The compound of claim 8, wherein the cyclic silicane of $(CH_2)_pT$ is unsaturated.

**10.** The compound of claim 1, wherein Q is -$CH_2$-phenylene and $R_1$, $R_2$ and $R_3$ are each $C_{1-7}$ alkyl.

**11.** The compound of claim 1, wherein -Q-Si-$R_1R_2R_3$ is selected from the following groups:
-$CH_2$-Si-$(CH_3)_3$,
-$(CH_2)_3$-Si$(CH_3)_3$,
-$(CH_2)_4$-Si$(CH_3)_3$,
-$CH_2Si(CH_3)_2C_6H_5$,
-$CH_2Si(CH_3)_2C_2H_5$,
-$CH_2Si(CH_3)_2C_3H_7$,
trans-$CH_2$-CH=CH-Si$(CH_3)_3$,
-$CH_2$-$C_6H_4$-meta-Si$(CH_3)_3$,
-$(CH_2)_3$-Si$(CH_3)_2CH_2OH$,
-$(CH_2)_3$-Si$(CH_3)_2CH_2CHOHCH_2OH$,

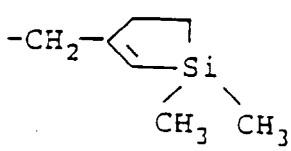

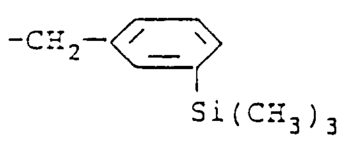

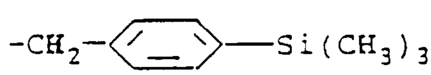

**12.** The compound of claim 1 further comprising a pharmaceutically acceptable carrier.

**13.** A method for preparing compounds of the formula

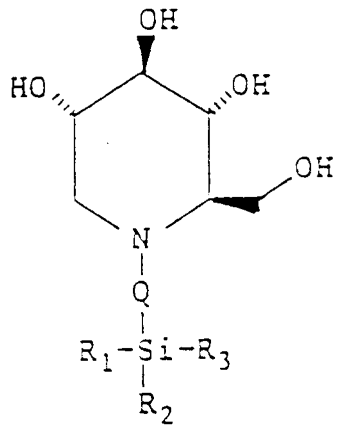

the geometric isomers thereof, and the pharmaceutically acceptable salts thereof wherein

Q is $C_{1-7}$ alkylene, $(CH_2)_mCH=CH(CH_2)_n$, $(CH_2)_mC\equiv C(CH_2)_n$, $(CH_2)_mCH=C=CH(CH_2)_n$, $(CH_2)_p$ phenylene, $(CH_2)_m$ cyclopentenylene, $(CH_2)_m$ cyclohexenylene, $(CH_2)_pT$, wherein T, together with the silicon atom to which it is attached forms a 5 to 6 atom cyclic silicane, said cyclic silicane optionally having a double bond, with m being 1, 2 or 3, n being 0, 1 or 2, p being 0, 1, 2, 3 or 4,

$R_1$ is $C_{1-7}$ alkyl, $C_{1-7}$ alkoxy, mono-or polyhydroxy $C_{1-6}$ alkyl, mono-or polyalkoxy $C_{1-6}$ alkyl, chloro $C_{1-6}$ alkyl,

$R_2$ and $R_3$ are $C_{1-10}$ alkyl, $(CH_2)_p$-X,Y-substituted phenyl, with X and Y each being H, OH, halogen, $C_{1-6}$ alkyl, $C_{1-6}$ alkoxy, $CF_3$, CN, $NO_2$, SH or -S-$C_{1-6}$ alkyl, with the proviso that when Q is $(CH_2)_pT$, then one of $R_1$, $R_2$ or $R_3$ is deleted which comprises condensing an optionally hydroxy-protected compound of the formula

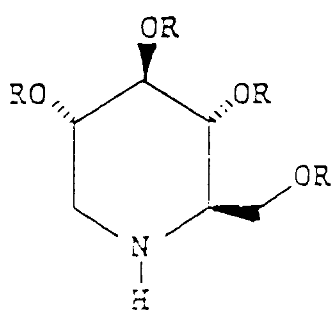

wherein R is H or an optional protecting group with a reactant X'-Q-$SiR_1R_2R_3$ wherein X' is halogeno, mesylate or tosylate followed by the removal of any hydroxy-protecting group and, if necessary, neutralizing any anion formed during the deprotection process.

**14.** Use of the compound according to claims 1 to 13 for the preparation of medicaments useful for the treatment of hyperglycemia.

**15.** Use of the compound according to claims 1 to 13 for the preparation of medicaments useful for the treatment of obesity associated with dietary improprieties.

**16.** Use of the compound according to claims 1 to 13 for the preparation of medicaments useful for the treatment of AIDS.

**Patentansprüche**

**Patentansprüche für folgende Vertragsstaaten : AT, BE, CH, DE, DK, FR, GB, IT, LI, LU, NL, SE**

1. Verbindung der Formel

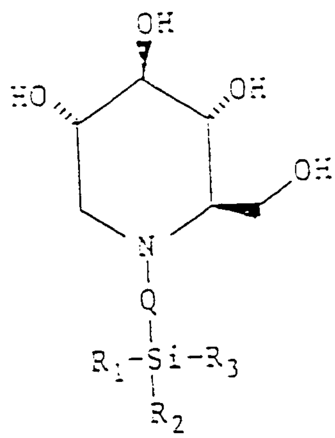

ihre geometrischen Isomeren und ihre pharmazeutisch verträglichen Salze, in der

Q einen $C_{1-7}$-Alkylenrest, einen Rest der Formeln $(CH_2)_mCH=CH(CH_2)_n$, $(CH_2)_mC{\equiv}C(CH_2)_n$ oder $(CH_2)_mCH=C=CH(CH_2)_n$, einen $(CH_2)_p$ Phenylen-, $(CH_2)_m$ Cyclopentenylen- oder $(CH_2)_m$ Cyclohexenylenrest oder einen Rest der Formel $(CH_2)_pT$ bedeutet, wobei T, zusammen mit dem Siliziumatom, an das es gebunden ist, eine cyclische Silizium-organische Verbindung mit 5 bis 6 Atomen bildet, wobei die cyclische Silizium-organische Verbindung gegebenenfalls eine Doppelbindung aufweist, wobei m 1, 2 oder 3 bedeutet, n 0, 1 oder 2 ist und p den Wert 0, 1, 2, 3 oder 4 hat,

$R_1$ einen $C_{1-7}$-Alkyl-, $C_{1-7}$-Alkoxy-, Mono- oder Polyhydroxy-$C_{1-6}$-alkyl-, Mono- oder Polyalkoxy-$C_{1-6}$-alkyl- oder Chlor-$C_{1-6}$-alkylrest bedeutet,

$R_2$ und $R_3$ einen $C_{1-10}$-Alkyl- oder $(CH_2)_p$-X,Y-substituierten Phenylrest bedeutet, wobei X und Y jeweils H, OH, Halogen, $C_{1-6}$-Alkyl, $C_{1-6}$-Alkoxy, $CF_3$, CN, $NO_2$, SH oder -S-$C_{1-6}$-Alkyl bedeuten, mit der Maßgabe, daß, wenn Q einen Rest der Formel $(CH_2)_pT$ bedeutet, einer der Reste $R_1$, $R_2$ oder $R_3$ gestrichen ist.

2. Verbindung nach Anspruch 1, wobei Q ein $C_{1-7}$-Alkylenrest ist.

3. Verbindung nach Anspruch 1, wobei Q ein Rest der Formel $(CH_2)_mHC=CH(CH_2)_n$ ist.

4. Verbindung nach Anspruch 2 oder 3, wobei $R_1$, $R_2$ und $R_3$ $C_{1-7}$-Alkylreste sind.

5. Verbindung nach Anspruch 2, wobei der $C_{1-7}$-Alkylrest eine Methylgruppe ist.

6. Verbindung nach Anspruch 2, wobei $R_1$ ein hydroxylierter $C_{1-7}$-Alkylrest ist.

7. Verbindung nach Anspruch 1, wobei Q ein Rest der Formel $(CH_2)_pT$ ist.

8. Verbindung nach Anspruch 7, wobei $R_1$ und $R_2$ $C_{1-7}$-Alkylreste sind und $R_3$ gestrichen ist.

9. Verbindung nach Anspruch 8, wobei die cyclische Silizium-organische Verbindung des Restes $(CH_2)_pT$ ungesättigt ist.

10. Verbindung nach Anspruch 1, wobei Q eine $-CH_2$-Phenylengruppe ist und $R_1$, $R_2$ und $R_3$ jeweils $C_{1-7}$-Alkylreste sind.

11. Verbindung nach Anspruch 1, wobei -Q-Si-$R_1R_2R_3$ aus den folgenden Gruppen ausgewählt ist: $-CH_2-Si-(CH_3)_3$, $-(CH_2)_3-Si(CH_3)_3$, $-(CH_2)_4-Si(CH_3)_3$, $-CH_2Si(CH_3)_2C_6H_5$, $-CH_2Si(CH_3)_2C_2H_5$, $-CH_2Si$-

$(CH_3)_2C_3H_7$, trans-$CH_2$-$CH=CH$-$Si(CH_3)_3$, -$CH_2$-$C_6H_4$-meta-$Si(CH_3)_3$, -$(CH_2)_3$-$Si(CH_3)_2CH_2OH$, -$(CH_2)_3$-$Si(CH_3)_2CH_2CHOHCH_2OH$,

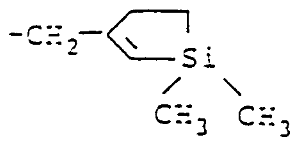

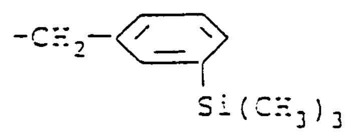

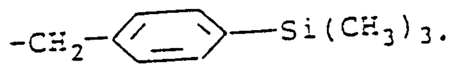

**12.** Verbindung nach Anspruch 1, weiter umfassend einen pharmazeutisch verträglichen Träger.

**13.** Verfahren zur Herstellung von Verbindungen der Formel

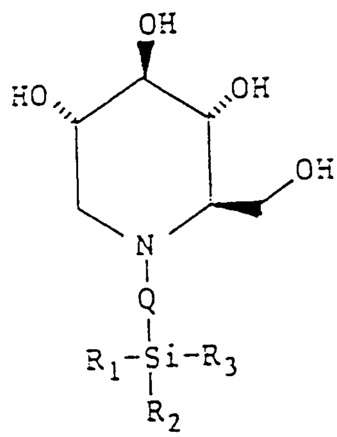

ihrer geometrischen Isomeren und ihrer pharmazeutisch verträglichen Salze, in der

| | |
|---|---|
| Q | einen $C_{1-7}$-Alkylenrest, einen Rest der Formeln $(CH_2)_mCH=CH(CH_2)_n$, $(CH_2)_mC{\equiv}C$-$(CH_2)_n$ oder $(CH_2)_mCH=C=CH(CH_2)_n$, einen $(CH_2)_p$ Phenylen-, $(CH_2)_m$ Cyclopentenylen- oder $(CH_2)_m$ Cyclohexenylenrest oder einen Rest der Formel $(CH_2)_pT$ bedeutet, wobei T, zusammen mit dem Siliziumatom, an das es gebunden ist, eine cyclische Silizium-organische Verbindung mit 5 bis 6 Atomen bildet, wobei die cyclische Silizium-organische Verbindung gegebenenfalls eine Doppelbindung aufweist, wobei m 1, 2 oder 3 bedeutet, n 0, 1 oder 2 ist und p den Wert 0, 1, 2, 3 oder 4 hat, |
| $R_1$ | einen $C_{1-7}$-Alkyl-, $C_{1-7}$-Alkoxy-, Mono- oder Polyhydroxy-$C_{1-6}$-alkyl-, Mono- oder Polyalkoxy-$C_{1-6}$-alkyl- oder Chlor-$C_{1-6}$-alkylrest bedeutet, |
| $R_2$ und $R_3$ | einen $C_{1-10}$-Alkyl- oder $(CH_2)_p$-X,Y-substituierten Phenylrest bedeutet, wobei X und Y jeweils H, OH, Halogen, $C_{1-6}$-Alkyl, $C_{1-6}$-Alkoxy, $CF_3$, CN, $NO_2$, SH oder -S-$C_{1-6}$-Alkyl bedeuten, mit der Maßgabe, daß, wenn Q einen Rest der Formel $(CH_2)_pT$ bedeutet, einer der Reste $R_1$, $R_2$ oder $R_3$ gestrichen ist, umfassend die Kondensation einer gegebenenfalls hydroxylgeschützten Verbindung der Formel |

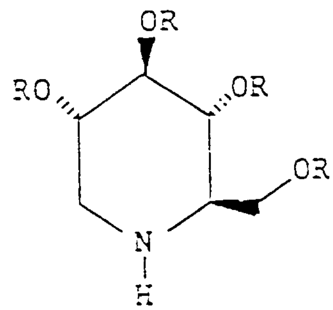

in der R ein Wasserstoffatom oder gegebenenfalls eine Schutzgruppe ist, mit einem Reaktionsteilnehmer X'-Q-$SiR_1R_2R_3$, in dem X' ein Halogenatom, eine Mesilat- oder Tosylatgruppe ist, gefolgt von der Entfernung irgendwelcher Hydroxylschutzgruppen, und, falls nötig, Neutralisieren irgendwelcher während der Schutzgruppenabspaltung gebildeter Anionen.

**14.** Verbindung nach Anspruch 1 zur Verwendung als pharmazeutischer Wirkstoff.

**15.** Verwendung der Verbindung nach den Ansprüchen 1 bis 13 zur Herstellung von Arzneimitteln, die sich zur Behandlung von Hyperglykämie eignen.

**16.** Verwendung der Verbindung nach den Ansprüchen 1 bis 13 zur Herstellung von Arzneimitteln, die sich zur Behandlung von mit Ernährungsstörungen verbundener Fettleibigkeit eignen.

**17.** Verwendung der Verbindung nach den Ansprüchen 1 bis 13 zur Herstellung von Arzneimitteln, die sich zur Behandlung von AIDS eignen.

**Patentansprüche für folgenden Vertragsstaat: ES**

**1.** Verfahren zur Herstellung einer Verbindung der Formel

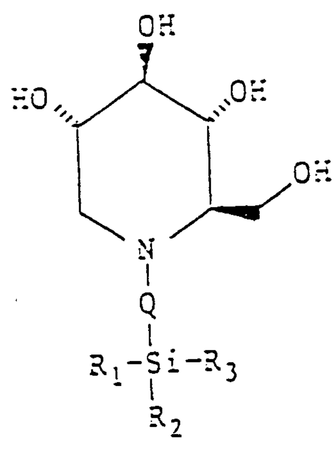

ihrer geometrischen Isomeren und ihrer pharmazeutisch verträglichen Salze, in der

Q einen $C_{1-7}$-Alkylenrest, einen Rest der Formeln $(CH_2)_mCH=CH(CH_2)_n$, $(CH_2)_mC{\equiv}C-(CH_2)_n$ oder $(CH_2)_mCH=C=CH(CH_2)_n$, einen $(CH_2)_p$ Phenylen-, $(CH_2)_m$ Cyclopentenylen- oder $(CH_2)_m$ Cyclohexenylenrest oder einen Rest der Formel $(CH_2)_pT$ bedeutet, wobei T, zusammen mit dem Siliziumatom, an das es gebunden ist, eine cyclische Silizium-organische Verbindung mit 5 bis 6 Atomen bildet, wobei die cyclische Silizium-organische Verbindung gegebenenfalls eine Doppelbindung aufweist, wobei m 1, 2 oder 3 bedeutet, n 0, 1 oder 2 ist und p den Wert 0, 1, 2, 3 oder 4 hat,

$R_1$ einen $C_{1-7}$-Alkyl-, $c_{1-7}$-Alkoxy-, Mono- oder Polyhydroxy-$C_{1-6}$-alkyl-, Mono- oder

Polyalkoxy-$C_{1-6}$-alkyl- oder Chlor-$C_{1-6}$-alkylrest bedeutet,

$R_2$ und $R_3$ einen $C_{1-10}$-Alkyl- oder $(CH_2)_p$-X,Y-substituierten Phenylrest bedeutet, wobei X und Y jeweils H, OH, Halogen, $C_{1-6}$-Alkyl, $C_{1-6}$-Alkoxy, $CF_3$, CN, $NO_2$, SH oder -S-$C_{1-6}$-Alkyl bedeuten, mit der Maßgabe, daß, wenn Q einen Rest der Formel $(CH_2)_pT$ bedeutet, einer der Reste $R_1$, $R_2$ oder $R_3$ gestrichen ist, umfassend die Kondensation einer gegebenenfalls hydroxylgeschützten Verbindung der Formel

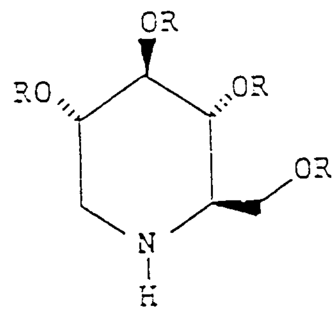

in der R ein Wasserstoffatom oder gegebenenfalls eine Schutzgruppe ist, mit einem Reaktionsteilnehmer X'-Q-$SiR_1R_2R_3$, in dem X' ein Halogenatom, eine Mesilat- oder Tosylatgruppe ist, gefolgt von der Entfernung irgendwelcher Hydroxylschutzgruppen, und, falls nötig, Neutralisieren irgendwelcher während der Schutzgruppenabspaltung gebildeter Anionen.

**2.** Verfahren nach Anspruch 1, wobei ein Reaktionsteilnehmer X'-Q-$SiR_1R_2R_3$ verwendet wird, in dem Q ein $C_{1-7}$-Alkylenrest ist, wobei eine Verbindung der Formel (I) erhalten wird, in der Q ein $C_1$-$C_7$-Alkylenrest ist.

**3.** Verfahren nach Anspruch 1, wobei ein Reaktionsteilnehmer X'-Q-$SiR_1R_2R_3$ verwendet wird, in dem Q ein Rest der Formel $(CH_2)_mHC=CH(CH_2)_n$ ist, wobei eine Verbindung der Formel (I) erhalten wird, in der Q $(CH_2)_mHC=CH(CH_2)_n$ ist.

**4.** Verfahren nach Anspruch 1, wobei ein Reaktionsteilnehmer X'-Q-$SiR_1R_2R_3$ verwendet wird, in dem $R_1$, $R_2$ und $R_3$ $C_{1-7}$-Alkylreste sind, wobei eine Verbindung der Formel (I) erhalten wird, in der $R_1$, $R_2$ und $R_3$ $C_{1-7}$-Alkylreste sind.

**5.** Verfahren nach Anspruch 4, wobei der $C_{1-7}$-Alkylrest eine Methylgruppe ist.

**6.** Verfahren nach Anspruch 1, wobei ein Reaktionsteilnehmer X'-Q-$SiR_1R_2R_3$ verwendet wird, in dem $R_1$ ein hydroxylierter $C_{1-7}$-Alkylrest ist, wobei eine Verbindung der Formel (I) erhalten wird, in der $R_1$ ein hydroxylierter $C_{1-7}$-Alkylrest ist.

**7.** Verfahren nach Anspruch 1, wobei ein Reaktionsteilnehmer X'-Q-$SiR_1R_2R_3$ verwendet wird, in dem Q ein Rest der Formel $(CH_2)_pT$ ist, wobei eine Verbindung der Formel (I) erhalten wird, in der Q ein Rest der Formel $(CH_2)_pT$ ist.

**8.** Verfahren nach Anspruch 1, wobei ein Reaktionsteilnehmer X'-Q-$SiR_1R_2R_3$ verwendet wird, in dem $R_1$ und $R_2$ $C_{1-7}$-Alkylreste sind und $R_3$ gestrichen ist, wobei eine Verbindung der Formel (I) erhalten wird, in der $R_1$ und $R_2$ $C_{1-7}$-Alkylreste sind und $R_3$ gestrichen ist.

**9.** Verfahren nach Anspruch 8, wobei ein Reaktionsteilnehmer X'-Q-$SiR_1R_2R_3$ verwendet wird, in dem Q ein Rest der Formel $(CH_2)_pT$ ist, in der T mit dem Siliziumatom, an das es gebunden ist, eine ungesättigte cyclische Siliziumorganische Verbindung bildet, wobei eine Verbindung der Formel (I) erhalten wird, in der der Rest $(CH_2)_pT$ eine ungesättigte cyclische Silizium-organische Verbindung ist.

**10.** Verfahren nach Anspruch 1, wobei ein Reaktionsteilnehmer X'-Q-$SiR_1R_2R_3$ verwendet wird, in dem Q eine -$CH_2$-Phenylengruppe ist und $R_1$, $R_2$ und $R_3$ jeweils $C_{1-7}$-Alkylreste sind, wobei eine Verbindung

der Formel (I) erhalten wird, in der Q eine $-CH_2$-Phenylengruppe ist und $R_1$, $R_2$ und $R_3$ jeweils $C_{1-7}$Alkylreste sind.

**11.** Verfahren nach Anspruch 1, wobei ein Reaktionsteilnehmer $X'$-Q-$SiR_1R_2R_3$ verwendet wird, in dem -Q-Si-$R_1R_2R_3$ aus den folgenden Gruppen ausgewählt ist:
$-CH_2-Si-(CH_3)_3$, $-(CH_2)_3-Si(CH_3)_3$, $-(CH_2)_4-Si(CH_3)_3$, $-CH_2Si(CH_3)_2C_6H_5$, $-CH_2Si(CH_3)_2C_2H_5$, $-CH_2Si(CH_3)_2C_3H_7$, trans-$CH_2-CH=CH-Si(CH_3)_3$, $-CH_2-C_6H_4$-meta-$Si(CH_3)_3$, $-(CH_2)_3-Si(CH_3)_2CH_2OH$, $-(CH_2)_3-Si(CH_3)_2CH_2CHOHCH_2OH$,

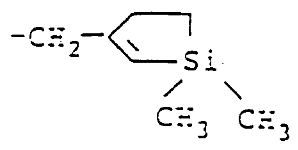

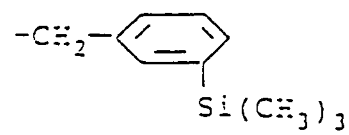

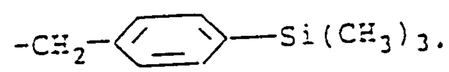

wobei eine Verbindung der Formel (I) erhalten wird, in der Q-$SiR_1R_2R_3$ die vorstehenden Bedeutungen hat.

**12.** Verwendung der nach einem der Ansprüche 1 bis 11 erhaltenen Verbindung zur Herstellung von Arzneimitteln, die sich zur Behandlung von Hyperglykämie eignen.

**13.** Verwendung der nach einem der Ansprüche 1 bis 11 erhaltenen Verbindung zur Herstellung von Arzneimitteln, die sich zur Behandlung von mit Ernährungsstörungen verbundener Fettleibigkeit eignen.

**14.** Verwendung der nach einem der Ansprüche 1 bis 11 erhaltenen Verbindung zur Herstellung von Arzneimitteln, die sich zur Behandlung von AIDS eignen.

**Patentansprüche für folgenden Vertragsstaat : ES**

**1.** Verbindung der Formel

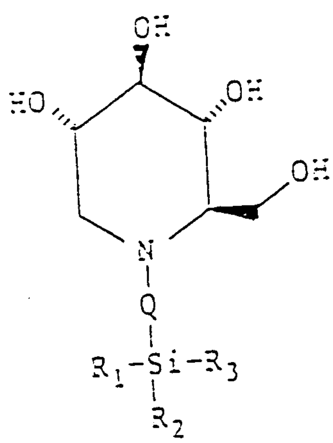

ihre geometrischen Isomeren und ihre pharmazeutisch verträglichen Salze, in der

Q einen $C_{1-7}$-Alkylenrest, einen Rest der Formeln $(CH_2)_mCH=CH(CH_2)n$, $(CH_2)_mC{\equiv}C-(CH_2)_n$ oder $(CH_2)_mCH=C=CH(CH_2)_n$, einen $(CH_2)_p$ Phenylen-, $(CH_2)_m$ Cyclopentenylen- oder $(CH_2)_m$ Cyclohexenylenrest oder einen Rest der Formel $(CH_2)_pT$ bedeutet, wobei T, zusammen mit dem Siliziumatom, an das es gebunden ist, eine cyclische

Silizium-organische Verbindung mit 5 bis 6 Atomen bildet, wobei die cyclische Silizium-organische Verbindung gegebenenfalls eine Doppelbindung aufweist, wobei m 1, 2 oder 3 bedeutet, n 0, 1 oder 2 ist und p den Wert 0, 1, 2, 3 oder 4 hat,

$R_1$ einen $C_{1-7}$-Alkyl-, $C_{1-7}$-Alkoxy-, Mono- oder Polyhydroxy-$C_{1-6}$-alkyl-, Mono- oder Polyalkoxy-$C_{1-6}$-alkyl- oder Chlor-$C_{1-6}$-alkylrest bedeutet,

$R_2$ und $R_3$ einen $C_{1-10}$-Alkyl- oder $(CH_2)_p$-X,Y-substituierten Phenylrest bedeutet, wobei X und Y jeweils H, OH, Halogen, $C_{1-6}$-Alkyl, $C_{1-6}$-Alkoxy, $CF_3$, CN, $NO_2$, SH oder -S-$C_{1-6}$-Alkyl bedeuten, mit der Maßgabe, daß, wenn Q einen Rest der Formel $(CH_2)_pT$ bedeutet, einer der Reste $R_1$, $R_2$ oder $R_3$ gestrichen ist.

**2.** Verbindung nach Anspruch 1, wobei Q ein $C_{1-7}$-Alkylenrest ist.

**3.** Verbindung nach Anspruch 1, wobei Q ein Rest der Formel $(CH_2)_mHC=CH(CH_2)_n$ ist.

**4.** Verbindung nach Anspruch 2 oder 3, wobei $R_1$, $R_2$ und $R_3$ $C_{1-7}$-Alkylreste sind.

**5.** Verbindung nach Anspruch 2, wobei der $C_{1-7}$-Alkylrest eine Methylgruppe ist.

**6.** Verbindung nach Anspruch 2, wobei $R_1$ ein hydroxylierter $C_{1-7}$-Alkylrest ist.

**7.** Verbindung nach Anspruch 1, wobei Q ein Rest der Formel $(CH_2)_pT$ ist.

**8.** Verbindung nach Anspruch 7, wobei $R_1$ und $R_2$ $C_{1-7}$-Alkyl-reste sind und $R_3$ gestrichen ist.

**9.** Verbindung nach Anspruch 8, wobei die cyclische Silizium-organische Verbindung des Restes $(CH_2)_pT$ ungesättigt ist.

**10.** Verbindung nach Anspruch 1, wobei Q eine -$CH_2$-Phenylengruppe ist und $R_1$, $R_2$ und $R_3$ jeweils $C_{1-7}$-Alkylreste sind.

**11.** Verbindung nach Anspruch 1, wobei -Q-Si-$R_1R_2R_3$ aus den folgenden Gruppen ausgewählt ist: -$CH_2$-Si-$(CH_3)_3$, -$(CH_2)_3$-Si$(CH_3)_3$, -$(CH_2)_4$-Si$(CH_3)_3$, -$CH_2$Si$(CH_3)_2C_6H_5$, -$CH_2$Si$(CH_3)_2C_2H_5$, -$CH_2$Si-$(CH_3)_2C_3H_7$,trans-$CH_2$-CH=CH-Si$(CH_3)_3$, -$CH_2$-$C_6H_4$-meta-Si$(CH_3)_3$, -$(CH_2)_3$-Si$(CH_3)_2CH_2$OH, -$(CH_2)_3$-Si$(CH_3)_2CH_2$CHOHCH$_2$OH,

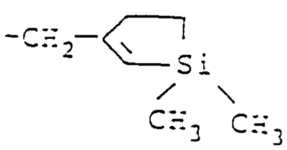

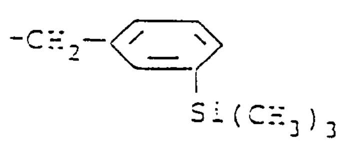

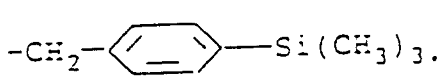

**12.** Verbindung nach Anspruch 1, weiter umfassend einen pharmazeutisch verträglichen Träger.

**13.** Verfahren zur Herstellung von Verbindungen der Formel

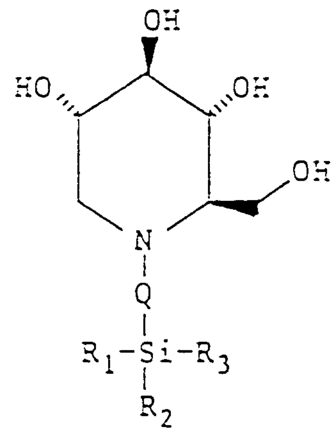

ihrer geometrischen Isomeren und ihrer pharmazeutisch verträglichen Salze, in der

Q einen $C_{1-7}$-Alkylenrest, einen Rest der Formeln $(CH_2)_mCH=CH(CH_2)_n$, $(CH_2)_mC{\equiv}C\text{-}(CH_2)_n$ oder $(CH_2)_mCH=C=CH(CH_2)_n$, einen $(CH_2)_p$ Phenylen-, $(CH_2)_m$ Cyclopentenylen oder $(CH_2)_m$ Cyclohexenylenrest oder einen Rest der Formel $(CH_2)pT$ bedeutet, wobei T, zusammen mit dem Siliziumatom, an das es gebunden ist, eine cyclische Silizium-organische Verbindung mit 5 bis 6 Atomen bildet, wobei die cyclische Silizium-organische Verbindung gegebenenfalls eine Doppelbindung aufweist, wobei m 1, 2 oder 3 bedeutet, n 0, 1 oder 2 ist und p den Wert 0, 1, 2, 3 oder 4 hat,

$R_1$ einen $C_{1-7}$-Alkyl-, $C_{1-7}$-Alkoxy-, Mono- oder Polyhydroxy-$C_{1-6}$-alkyl-, Mono- oder Polyalkoxy-$C_{1-6}$-alkyl- oder Chlor-$C_{1-6}$-alkylrest bedeutet,

$R_2$ und $R_3$ einen $C_{1-10}$-Alkyl- oder $(CH_2)_p$-X,Y-substituierten Phenylrest bedeutet, wobei X und Y jeweils H, OH, Halogen, $C_{1-6}$-Alkyl, $C_{1-6}$-Alkoxy, $CF_3$, CN, $NO_2$, SH oder $\text{-S-}C_{1-6}$-Alkyl bedeuten, mit der Maßgabe, daß, wenn Q einen Rest der Formel $(CH_2)_pT$ bedeutet, einer der Reste $R_1$, $R_2$ oder $R_3$ gestrichen ist, umfassend die Kondensation einer gegebenenfalls hydroxylgeschützten Verbindung der Formel

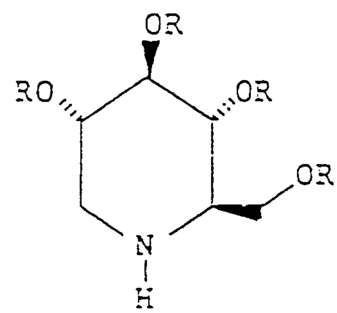

in der R ein Wasserstoffatom oder gegebenenfalls eine Schutzgruppe ist, mit einem Reaktionsteilnehmer $X'\text{-Q-SiR}_1R_2R_3$, in dem X' ein Halogenatom, eine Mesilat- oder Tosylatgruppe ist, gefolgt von der Entfernung irgendwelcher Hydroxylschutzgruppen, und, falls nötig, Neutralisieren irgendwelcher während der Schutzgruppenabspaltung gebildeter Anionen.

**14.** Verwendung der Verbindung nach den Ansprüchen 1 bis 13 zur Herstellung von Arzneimitteln, die sich zur Behandlung von Hyperglykämie eignen.

**15.** Verwendung der Verbindung nach den Ansprüchen 1 bis 13 zur Herstellung von Arzneimitteln, die sich zur Behandlung von mit Ernährungsstörungen verbundener Fettleibigkeit eignen.

16. Verwendung der Verbindung nach den Ansprüchen 1 bis 13 zur Herstellung von Arzneimitteln, die sich zur Behandlung von AIDS eignen.

**Revendications**
**Revendications pour les Etats contractants suivants : AT, BE, CH, DE, DK, FR, GB, IT, LI, LU, NL, SE**

1. Composé de formule

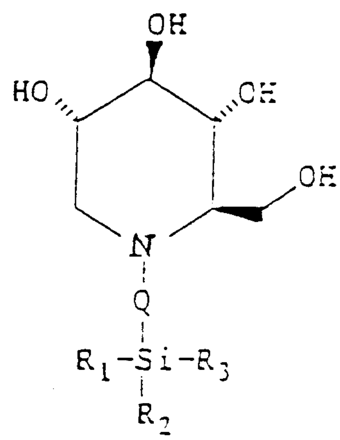

ses isomères géométriques et ses sels pharmaceutiquement acceptables, dans laquelle

Q représente un $C_{1-7}$ alkylène, $(CH_2)_mCH=CH(CH_2)_n$, $(CH_2)_mC\equiv C(CH_2)_n$, $(CH_2)_mCH=C=CH(CH_2)_n$, un $(CH_2)_p$ phénylène, un $(CH_2)_m$ cyclopenténylène, un $(CH_2)_m$ cyclohexénylène, $(CH_2)_pT$ pour lequel T, avec l'atome de silicium auquel il est lié, forme un silicane cyclique ayant 5 ou 6 atomes, ledit silicane cyclique ayant éventuellement une double liaison, m représentant 1, 2 ou 3, n représentant 0, 1 ou 2, p représentant 0, 1, 2, 3 ou 4,

$R_1$ représente un $C_{1-7}$ alkyle, un $C_{1-7}$ alcoxy, un $C_{1-6}$ alkyle monohydroxylé ou polyhydroxylé, un $C_{1-6}$ alkyle monoalcoxylé ou polyalcoxylé, un $C_{1-6}$ alkyle chloré,

$R_2$ et $R_3$ représentent un $C_{1-10}$ alkyle, un $(CH_2)_p$-phényle X,Y-substitué, X et Y représentant chacun H, OH, un atome d'halogène, un $C_{1-6}$ alkyle, un $C_{1-6}$ alcoxy, $CF_3$, CN, $NO_2$, SH ou un -S-$C_{1-6}$ alkyle, à condition que lorsque Q représente $(CH_2)_pT$, un radical parmi $R_1$, $R_2$ et $R_3$ est supprimé.

2. Composé selon la revendication 1, pour lequel Q représente un $C_{1-7}$ alkylène.

3. Composé selon la revendication 1, pour lequel Q représente $(CH_2)_mHC=CH(CH_2)_n$.

4. Composé selon la revendication 2 ou 3, pour lequel $R_1$, $R_2$ et $R_3$ représentent un $C_{1-7}$ alkyle.

5. Composé selon la revendication 2, pour lequel le $C_{1-7}$ alkyle est le méthyle.

6. Composé selon la revendication 2, pour lequel $R_1$ représente un $C_{1-7}$ alkyle hydroxylé.

7. Composé selon la revendication 1, pour lequel Q représente $(CH_2)_pT$.

8. Composé selon la revendication 7, pour lequel $R_1$ et $R_2$ représentent un $C_{1-7}$ alkyle et $R_3$ est supprimé.

9. Composé selon la revendication 8, pour lequel le silicane cyclique de $(CH_2)_pT$ est insaturé.

10. Composé selon la revendication 1, pour lequel Q représente un -$CH_2$-phénylène et $R_1$, $R_2$ et $R_3$ représentent chacun un $C_{1-7}$ alkyle.

**11.** Composé selon la revendication 1, pour lequel on choisit -Q-Si-$R_1R_2R_3$ parmi les groupements suivants :

$-CH_2-Si-(CH_3)_3$,
$-(CH_2)_3-Si(CH_3)_3$,
$-(CH_2)_4-Si(CH_3)_3$,
$-CH_2Si(CH_3)_2C_6H_5$,
$-CH_2Si(CH_3)_2C_2H_5$,
$-CH_2Si(CH_3)_2C_3H_7$,
trans-$CH_2-CH=CH-Si(CH_3)_3$,
$-CH_2-C_6H_4$-méta-$Si(CH_3)_3$,
$-(CH_2)_3-Si(CH_3)_2CH_2OH$,
$-(CH_2)_3-Si(CH_3)_2CH_2CHOHCH_2OH$,

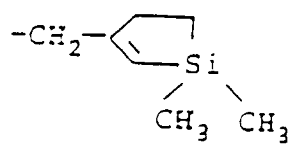

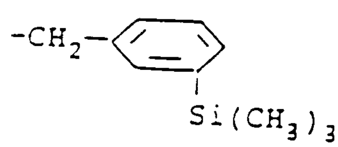

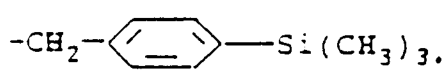

**12.** Composé selon la revendication 1, comprenant de plus un support pharmaceutiquement acceptable.

**13.** Procédé de préparation de composés de formule

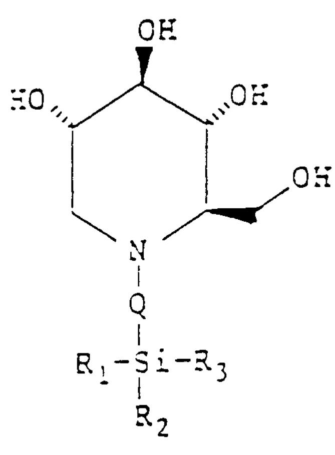

de leurs isomères géométriques et de leurs sels pharmaceutiquement acceptables, dans laquelle

Q représente un $C_{1-7}$ alkylène, $(CH_2)_mCH=CH(CH_2)_n$, $(CH_2)_mC\equiv C(CH_2)_n$, $(CH_2)_mCH=C=CH(CH_2)_n$, un $(CH_2)_p$ phénylène, un $(CH_2)_m$ cyclopenténylène, un $(CH_2)_m$

cyclohexénylène, $(CH_2)_pT$ pour lequel T, avec l'atome de silicium auquel il est lié, forme un silicane cyclique ayant 5 ou 6 atomes, ledit silicane cyclique ayant éventuellement une double liaison, m représentant 1, 2 ou 3, n représentant 0, 1 ou 2, p représentant 0, 1, 2, 3 ou 4,

$R_1$ représente un $C_{1-7}$ alkyle, un $C_{1-7}$ alcoxy, un $C_{1-6}$ alkyle monohydroxylé ou polyhydroxylé, un $C_{1-6}$ alkyle monoalcoxylé ou polyalcoxylé, un $C_{1-6}$ alkyle chloré,

$R_2$ et $R_3$ représentent un $C_{1-10}$ alkyle, un $(CH_2)_p$-phényle X,Y-substitué, X et Y représentant chacun H, OH, un atome d'halogène, un $C_1$-$C_6$ alkyle, un $C_{1-6}$ alcoxy, $CF_3$, CN, $NO_2$, SH ou un -S-$C_{1-6}$ alkyle, à condition que lorsque Q représente $(CH_2)_pT$, un radical parmi $R_1$, $R_2$ et $R_3$ est supprimé, lequel comprend la condensation d'un composé éventuellement à protection hydroxyle de formule

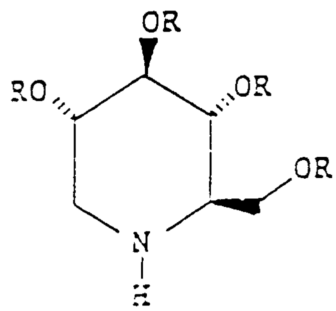

dans laquelle R représente H ou un groupement de protection éventuel, avec un corps réagissant X'-Q-$SiR_1R_2R_3$ pour lequel X' représente une fraction halogéno, mésylate ou tosylate, suivie de l'élimination de tout groupement de protection d'un hydroxyle et, si nécessaire, de la neutralisation de tout anion formé lors du procédé de déprotection.

**14.** Composé selon la revendication 1, à utiliser en tant qu'agent pharmaceutiquement actif.

**15.** Utilisation du composé selon les revendications 1 à 13 pour la préparation de médicaments utiles pour le traitement de l'hyperglycémie.

**16.** Utilisation du composé selon les revendications 1 à 13 pour la préparation de médicaments utiles pour le traitement de l'obésité associée aux mauvaises habitudes alimentaires.

**17.** Utilisation du composé selon les revendications 1 à 13 pour la préparation de médicaments utiles pour le traitement du SIDA.

**Revendications pour l'Etat contractant suivant : ES**

1. Procédé d'obtention d'un composé de formule

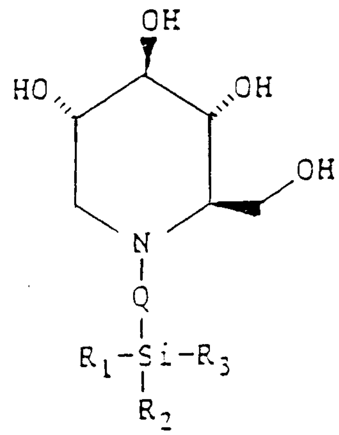

de ses isomères géométriques et de ses sels pharmaceutiquement acceptables, dans laquelle

Q représente un $C_{1-7}$ alkylène, $(CH_2)_mCH=CH(CH_2)_n$, $(CH_2)_mC{\equiv}C(CH_2)_n$, $(CH_2)_mCH=C=CH(CH_2)_n$, un $(CH_2)_p$ phénylène, un $(CH_2)_m$ cyclopenténylène, un $(CH_2)_m$ cyclohexénylène, $(CH_2)_pT$ pour lequel T, avec l'atome de silicium auquel il est lié, forme un silicane cyclique ayant 5 ou 6 atomes, ledit silicane cyclique ayant éventuellement une double liaison, m représentant 1, 2 ou 3, n représentant 0, 1 ou 2, p représentant 0, 1, 2, 3 ou 4,

$R_1$ représente un $C_{1-7}$ alkyle, un $C_{1-7}$ alcoxy, un $C_{1-6}$ alkyle monohydroxylé ou polyhydroxylé, un $C_{1-6}$ alkyle monoalcoxylé ou polyalcoxylé, un $C_{1-6}$ alkyle chloré,

$R_2$ et $R_3$ représentent un $C_{1-10}$ alkyle, un $(CH_2)_p$-phényle X,Y-substitué, X et Y représentant chacun H, OH, un atome d'halogène, un $C_{1-6}$ alkyle, un $C_{1-6}$ alcoxy, $CF_3$, CN, $NO_2$, SH ou un -S-$C_{1-6}$ alkyle, à condition que lorsque Q représente $(CH_2)_pT$, un radical parmi $R_1$, $R_2$ et $R_3$ est supprimé, lequel comprend la condensation d'un composé éventuellement à protection hydroxyle de formule

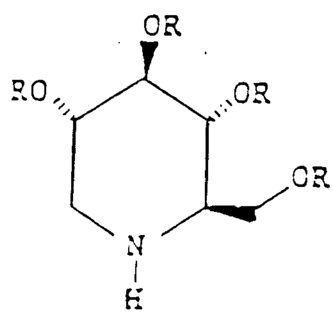

dans laquelle R représente H ou un groupement de protection éventuel, avec un corps réagissant X'-Q-$SiR_1R_2R_3$ pour lequel X' représente une fraction halogéno, mésylate ou tosylate, suivie de l'élimination de tout groupement de protection d'un hydroxyle et, si nécessaire, de la neutralisation de tout anion formé lors du procédé de déprotection.

2. Procédé selon la revendication 1, dans lequel on utilise un corps réagissant X'-Q-$SiR_1R_2R_3$, pour lequel Q représente un $C_{1-7}$ alkylène, pour obtenir un composé de formule (I) dans laquelle Q représente un $C_{1-7}$ alkylène.

3. Procédé selon la revendication 1, dans lequel on utilise un corps réagissant X'-Q-$SiR_1R_2R_3$, pour lequel Q représente $(CH_2)_mHC=CH(CH_2)_n$, pour obtenir un composé de formule (I) dans laquelle Q représente

$(CH_2)_mHC=CH(CH_2)_n$.

**4.** Procédé selon la revendication 1, dans lequel on utilise un corps réagissant X'-Q-$SiR_1R_2R_3$, pour lequel $R_1$, $R_2$ et $R_3$ représentent un $C_{1-7}$ alkyle, pour obtenir un composé de formule (I) dans laquelle $R_1$, $R_2$ et $R_3$ représentent un $C_{1-7}$ alkyle.

**5.** Procédé selon la revendication 4, dans lequel le $C_{1-7}$ alkyle est le méthyle.

**6.** Procédé selon la revendication 1, dans lequel on utilise un corps réagissant X'-Q-$SiR_1R_2R_3$, pour lequel $R_1$ représente un $C_{1-7}$ alkyle hydroxylé, pour obtenir un composé de formule (I) dans laquelle $R_1$ représente un $C_{1-7}$ alkyle hydroxylé.

**7.** Procédé selon la revendication 1, dans lequel on utilise un corps réagissant X'-Q-$SiR_1R_2R_3$, pour lequel Q représente $(CH_2)_pT$, pour obtenir un composé de formule (I) dans laquelle Q représente $(CH_2)_pT$.

**8.** Procédé selon la revendication 1, dans lequel on utilise un corps réagissant X'-Q-$SiR_1R_2R_3$, pour lequel $R_1$ et $R_2$ représentent un $C_{1-7}$ alkyle et $R_3$ est supprimé, pour obtenir un composé de formule (I) dans laquelle $R_1$ et $R_2$ représentent un $C_{1-7}$ alkyle et $R_3$ est supprimé.

**9.** Procédé selon la revendication 8, dans lequel on utilise un corps réagissant X'-Q-$SiR_1R_2R_3$, pour lequel Q représente $(CH_2)_pT$ pour lequel T, avec l'atome de silicium auquel il est fixé, forme un silicane cyclique insaturé, pour former un composé de formule (I) dans laquelle $(CH_2)_pT$ représente un silicane cyclique insaturé.

**10.** Procédé selon la revendication 1, dans lequel on utilise un corps réagissant X'-Q-$SiR_1R_2R_3$, pour lequel Q représente -$CH_2$-phénylène et $R_1$, $R_2$ et $R_3$ représentent chacun un $C_{1-7}$ alkyle, pour obtenir un composé de formule (I) dans laquelle Q représente -$CH_2$-phénylène et $R_1$, $R_2$ et $R_3$ représentent chacun un $C_{1-7}$ alkyle.

**11.** Procédé selon la revendication 1, dans lequel on utilise un corps réagissant X'-Q-$SiR_1R_2R_3$, pour lequel on choisit Q-$SiR_1R_2R_3$ parmi les groupements suivants :
-$CH_2$-Si-$(CH_3)_3$,
-$(CH_2)_3$-$Si(CH_3)_3$,
-$(CH_2)_4$-$Si(CH_3)_3$,
-$CH_2Si(CH_3)_2C_6H_5$,
-$CH_2Si(CH_3)_2C_2H_5$,
-$CH_2Si(CH_3)_2C_3H_7$,
trans-$CH_2$-CH=CH-$Si(CH_3)_3$,
-$CH_2$-$C_6H_4$-méta-$Si(CH_3)_3$,
-$(CH_2)_3$-$Si(CH_3)_2CH_2OH$,
-$(CH_2)_3$-$Si(CH_3)_2CH_2CHOHCH_2OH$,

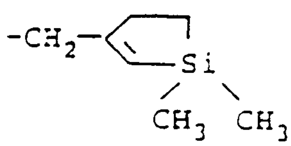

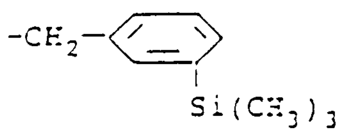

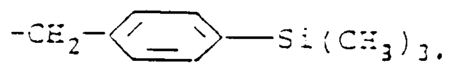

afin d'obtenir un composé de formule (I) dans laquelle Q-$SiR_1R_2R_3$ possède les significations ci-dessus.

**12.** Utilisation du composé obtenu selon le procédé de l'une quelconque des revendications 1 à 11 pour la préparation de médicaments utiles pour le traitement de l'hyperglycémie.

**13.** Utilisation du composé obtenu selon le procédé de l'une quelconque des revendications 1 à 11 pour la préparation de médicaments utiles pour le traitement de l'obésité associée aux mauvaises habitudes alimentaires.

**14.** Utilisation du composé obtenu selon le procédé de l'une quelconque des revendications 1 à 11 pour la préparation de médicaments utiles pour le traitement du SIDA.

**Revendications pour l'Etat contractant suivant : GR**

**1.** Composé de formule

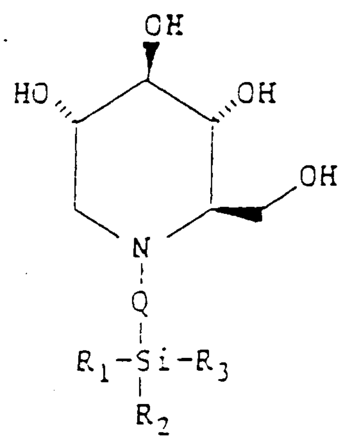

ses isomères géométriques et ses sels pharmaceutiquement acceptables, dans laquelle

- Q représente un $C_{1-7}$ alkylène, $(CH_2)_mCH=CH(CH_2)_n$, $(CH_2)_mC\equiv C(CH_2)_n$, $(CH_2)_mCH=C=CH(CH_2)_n$, un $(CH_2)_p$ phénylène, un $(CH_2)_m$ cyclopenténylène, un $(CH_2)_m$ cyclohexénylène, $(CH_2)_pT$ pour lequel T, avec l'atome de silicium auquel il est lié, forme un silicane cyclique ayant 5 ou 6 atomes, ledit silicane cyclique ayant éventuellement une double liaison, m représentant 1, 2 ou 3, n représentant 0, 1 ou 2, p représentant 0,1, 2, 3 ou 4,
- $R_1$ représente un $C_{1-7}$ alkyle, un $C_{1-7}$ alcoxy, un $C_{1-6}$ alkyle monohydroxylé ou polyhydroxylé, un $C_{1-6}$ alkyle monoalcoxylé ou polyalcoxylé, un $C_{1-6}$ alkyle chloré,
- $R_2$ et $R_3$ représentent un $C_{1-10}$ alkyle, un $(CH_2)_p$-phényle X,Y-substitué, X et Y représentant chacun H, OH, un atome d'halogène, un $C_{1-6}$ alkyle, un $C_{1-6}$ alcoxy, $CF_3$, CN, $NO_2$, SH ou un -S-$C_{1-6}$ alkyle, à condition que lorsque Q représente $(CH_2)_pT$, un radical parmi $R_1$, $R_2$ et $R_3$ est supprimé.

**2.** Composé selon la revendication 1, pour lequel Q représente un $C_{1-7}$ alkylène.

**3.** Composé selon la revendication 1, pour lequel Q représente $(CH_2)_mHC=CH(CH_2)_n$.

**4.** Composé selon la revendication 2 ou 3, pour lequel $R_1$, $R_2$ et $R_3$ représentent un $C_{1-7}$ alkyle.

**5.** Composé selon la revendication 2, pour lequel le $C_{1-7}$ alkyle est le méthyle.

**6.** Composé selon la revendication 2, pour lequel $R_1$ représente un $C_{1-7}$ alkyle hydroxylé.

**7.** Composé selon la revendication 1, pour lequel Q représente $(CH_2)_pT$.

**8.** Composé selon la revendication 7, pour lequel $R_1$ et $R_2$ représentent un $C_{1-7}$ alkyle et $R_3$ est supprimé.

**9.** Composé selon la revendication 8, pour lequel le silicane cyclique de $(CH_2)_pT$ est insaturé.

**10.** Composé selon la revendication 1, pour lequel Q représente un $-CH_2$-phénylène et $R_1$, $R_2$ et $R_3$ représentent chacun un $C_{1-7}$ alkyle.

**11.** Composé selon la revendication 1, pour lequel on choisit $-Q-Si-R_1R_2R_3$ parmi les groupements suivants :

$-CH_2-Si-(CH_3)_3$,
$-(CH_2)_3-Si(CH_3)_3$,
$-(CH_2)_4-Si(CH_3)_3$,
$-CH_2Si(CH_3)_2C_6H_5$,
$-CH_2Si(CH_3)_2C_2H_5$,
$-CH_2Si(CH_3)_2C_3H_7$,
trans-$CH_2-CH=CH-Si(CH_3)_3$,
$-CH_2-C_6H_4$-méta-$Si(CH_3)_3$,
$-(CH_2)_3-Si(CH_3)_2CH_2OH$,
$-(CH_2)_3-Si(CH_3)_2CH_2CHOHCH_2OH$,

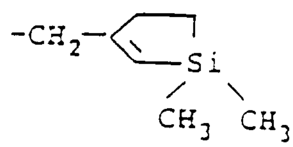

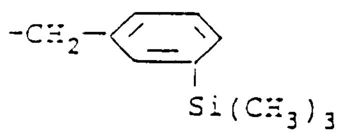

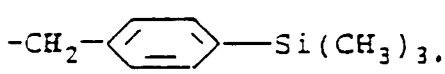

**12.** Composé selon la revendication 1, comprenant de plus un support pharmaceutiquement acceptable.

**13.** Procédé de préparation de composés de formule

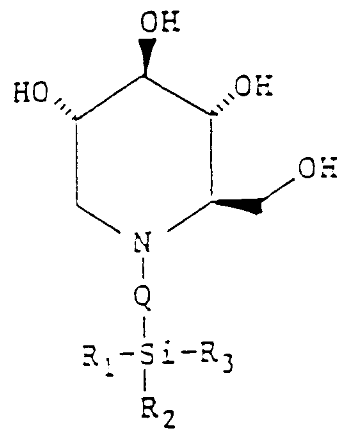

de leurs isomères géométriques et de leurs sels pharmaceutiquement acceptables, dans laquelle

Q représente un $C_{1-7}$ alkylène, $(CH_2)_mCH=CH(CH_2)_n$, $(CH_2)_mC\equiv C(CH_2)_n$, $(CH_2)_mCH=C=CH(CH_2)_n$, un $(CH_2)_p$ phénylène, un $(CH_2)_m$ cyclopenténylène, un $(CH_2)_m$ cyclohexénylène, $(CH_2)_pT$ pour lequel T, avec l'atome de silicium auquel il est lié, forme un silicane cyclique ayant 5 ou 6 atomes, ledit silicane cyclique ayant éventuellement une double liaison, m représentant 1, 2 ou 3, n représentant 0, 1 ou 2, p représentant 0, 1, 2, 3 ou 4,

$R_1$ représente un $C_{1-7}$ alkyle, un $C_{1-7}$ alcoxy, un $C_{1-6}$ alkyle monohydroxylé ou polyhydroxylé, un $C_{1-6}$ alkyle monoalcoxylé ou polyalcoxylé, un $C_{1-6}$ alkyle chloré,

$R_2$ et $R_3$ représentent un $C_{1-10}$ alkyle, un $(CH_2)_p$-phényle X,Y-substitué, X et Y représentant chacun H, OH, un atome d'halogène, un $C_{1-6}$ alkyle, un $C_{1-6}$ alcoxy, $CF_3$, CN, $NO_2$, SH ou un $-S-C_{1-6}$ alkyle, à condition que lorsque Q représente $(CH_2)_pT$, un radical parmi $R_1$, $R_2$ et $R_3$ est supprimé, lequel comprend la condensation d'un composé éventuellement à protection hydroxyle de formule

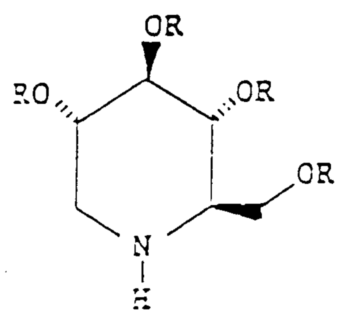

dans laquelle R représente H ou un groupement de protection éventuel, avec un corps réagissant $X'-Q-SiR_1R_2R_3$ pour lequel X' représente une fraction halogéno, mésylate ou tosylate, suivie de l'élimination de tout groupement de protection d'un hydroxyle et, si nécessaire, de la neutralisation de tout anion formé lors du procédé de déprotection.

**14.** Utilisation du composé selon les revendications 1 à 13 pour la préparation de médicaments utiles pour le traitement de l'hyperglycémie.

**15.** Utilisation du composé selon les revendications 1 à 13 pour la préparation de médicaments utiles pour le traitement de l'obésité associée aux mauvaises habitudes alimentaires.

**16.** Utilisation du composé selon les revendications 1 à 13 pour la préparation de médicaments utiles pour le traitement du SIDA.